# EUROPEAN PATENT APPLICATION

(11) **EP 3 964 576 A1**
(43) Date of publication of application: **09.03.2022**
(21) Application number: 20194571.4
(22) Date of filing: 04.09.2020
(51) Int. Cl.: C12N 15/10, A61K 48/00, A61P 31/14, C12N 15/67

(54) **MODIFIED MRNAS FOR VACCINE DEVELOPMENT**

(71) Applicant: baseclick GmbH, 82061 Neuried (DE)
(72) Inventor: The designation of the inventor has not yet been filed
(74) Representative: Böhm, Brigitte

(57) **Abstract**

A modified messenger RNA (mRNA) of the present invention encodes within its ORF an antigen such as a viral protein, an immunogenically active part of such viral protein, or an anticancer protein or epitope, and contains at least one of an alkyne- or azide-modification in at least one nucleotide. A preferred viral protein encoded by the inventive mRNA is a Corona virus protein, especially nucleoprotein N of SARS-CoV-2. The modified mRNA or a pharmaceutical composition containing such mRNA is especially useful in the context of a method for vaccination against viral infection and adding an adjuvant like a STING antagonist further enhances the immune response in an individual and accordingly the vaccination efficiency.

SEQUENCE LISTING

<110>  baseclick GmbH

<120>  Modified mRNAs for Vaccine Development

<130>  70578P EP-1

<140>  TBD
<141>  TBD

<160>  16

<170>  PatentIn version 3.5

<210>  1
<211>  33
<212>  DNA
<213>  artificial sequence

<220>
<223>  primer 1 plasmid amplification

<400>  1
ttcgccctat agtgagtcgt attaattaat aac                                    33


<210>  2
<211>  31
<212>  DNA
<213>  artificial sequence

<220>
<223>  primer 2 plasmid amplification

<400>  2
tcgaattgat ccagatctta agtaagtaag c                                      31


<210>  3
<211>  38
<212>  DNA
<213>  artificial sequence

<220>
<223>  Nucleocapside primer 1

<400>  3
cgactcacta tagggcgaac tagtaagcaa ggaggcgt                               38


<210>  4
<211>  43
<212>  DNA
<213>  artificial sequence

<220>
<223>  Nucleocapside primer 2

<400>  4
acttaagatc tggatcaatt cgattaggcc tgagttgagt cag                         43

<210>  5
<211>  37
<212>  DNA
<213>  artificial sequence

<220>
<223>  Envelope primer 1

<400>  5
cgactcacta tagggcgaac tagtaagcaa ggaggcg                                37


<210>  6
<211>  45
<212>  DNA
<213>  artificial sequence

<220>
<223>  Envelope primer 2

<400>  6
acttaagatc tggatcaatt cgattagacc agaagatcag gaact                       45


<210>  7
<211>  1282
<212>  DNA
<213>  SARS-CoV-2

<400>  7
ctagtaagca aggaggcgtg cagatgtctg ataatggacc ccaaaatcag cgaaatgcac       60

cccgcattac gtttggtgga ccctcagatt caactggcag taaccagaat ggagaacgca      120

gtggggcgcg atcaaaacaa cgtcggcccc aaggtttacc caataatact gcgtcttggt      180

tcaccgctct cactcaacat ggcaaggaag accttaaatt ccctcgagga caaggcgttc      240

caattaacac caatagcagt ccagatgacc aaattggcta ctaccgaaga gctaccagac      300

gaattcgtgg tggtgacggt aaaatgaaag atctcagtcc aagatggtat ttctactacc      360

taggaactgg gccagaagct ggacttccct atggtgctaa caaagacggc atcatatggg      420

ttgcaactga gggagccttg aatacaccaa aagatcacat tggcacccgc aatcctgcta      480

acaatgctgc aatcgtgcta caacttcctc aaggaacaac attgccaaaa ggcttctacg      540

cagaagggag cagaggcggc agtcaagcct cttctcgttc ctcatcacgt agtcgcaaca      600

gttcaagaaa ttcaactcca ggcagcagta ggggaacttc tcctgctaga atggctggca      660

atggcggtga tgctgctctt gctttgctgc tgcttgacag attgaaccag cttgagagca      720

aaatgtctgg taaaggccaa caacaacaag gccaaactgt cactaagaaa tctgctgctg      780

aggcttctaa gaagcctcgg caaaaacgta ctgccactaa agcatacaat gtaacacaag      840

ctttcggcag acgtggtcca gaacaaaccc aaggaaattt tggggaccag gaactaatca      900

gacaaggaac tgattacaaa cattggccgc aaattgcaca atttgccccc agcgcttcag      960

cgttcttcgg aatgtcgcgc attggcatgg aagtcacacc ttcgggaacg tggttgacct     1020

acacaggtgc catcaaattg gatgacaaag atccaaattt caaagatcaa gtcattttgc 1080

tgaataagca tattgacgca tacaaaacat tcccaccaac agagcctaaa aaggacaaaa 1140

agaagaaggc tgatgaaact cagccttacc gcagagacag aagaaacagc aaactgtgac 1200

tcttcttcct gctgcagatt tggatgattt ctccaaacaa ttgcaacaat ccatgagcag 1260

tgctgactca actcaggcct aa 1282

<210> 8
<211> 251
<212> DNA
<213> SARS-CoV-2

<400> 8
ctagtaagca aggaggcgtg cagatgtact cattcgtttc ggaagagaca ggtacgttaa 60

tagttaatag cgtacttctt tttcttgctt tcgtggtatt cttgctagtt acactagcca 120

tccttactgc gcttcgattg tgtgcgtact gctgcaatat tgttaacgtg agtcttgtaa 180

aaccttcttt ttacgtttac tctcgtgtta aaaatctgaa ttcttctaga gttcctgatc 240

ttctggtcta a 251

<210> 9
<211> 18
<212> DNA
<213> artificial sequence

<220>
<223> Envelope Sdm 1 Fwd

<400> 9
cagtacgcac acaatcga 18

<210> 10
<211> 21
<212> DNA
<213> artificial sequence

<220>
<223> Envelope Sdm 1 Rev

<400> 10
ctgcaatatt gttaacgtga g 21

<210> 11
<211> 23
<212> DNA
<213> artificial sequence

<220>
<223> Nucleocapsid 1 Fwd

<400> 11
aggtagtaga aataccatct tgc 23

<210>  12
<211>  17
<212>  DNA
<213>  artificial sequence

<220>
<223>  Nucleocapsid 1 Rev

<400>  12
aggaactggg ccagaag                                                      17


<210>  13
<211>  20
<212>  DNA
<213>  artificial sequence

<220>
<223>  Nucleocapsid 2 Fwd

<400>  13
tgagtttcat cagccttctt                                                   20


<210>  14
<211>  17
<212>  DNA
<213>  artificial sequence

<220>
<223>  Nucleocapsid 2 Rev

<400>  14
agccttaccg cagagac                                                      17


<210>  15
<211>  21
<212>  DNA
<213>  artificial sequence

<220>
<223>  Nucleocapsid 3 Fwd

<400>  15
taccgacgag ctaccagacg a                                                 21


<210>  16
<211>  24
<212>  DNA
<213>  artificial sequence

<220>
<223>  Nucleocapsid 3 Rev

<400>  16
gtagccaatt tggtcatctg gact                                              24

## Description

The present invention relates to an alkyne- and/or azide-modified messenger RNA (mRNA) which encodes an antigen such as a viral protein within its ORF, an immunogenically active part of such viral protein or an anticancer protein or epitope. The invention further relates to a process for producing such modified mRNA and kits for production and/or delivery of the modified mRNA, to pharmaceutical compositions comprising modified mRNA as well as to uses of such modified mRNAs. A method for vaccination against an antigen, especially viral infection such as the one caused by SARS-CoV-2 is a further, especially important aspect of the present invention. A second, important part of the vaccination method is the use an immune response stimulating agent in particular an agonist to stimulate the Stimulator of interferon genes (STING) receptor.

### Background of the invention

The current problem with corona virus epidemics/pandemics is the high variability of the surface proteins (Spike proteins (S) and Membrane glycoprotein (M)) which leads to variations even in the entry mechanism and binding to surface receptors. Secondly, the high mutation rate or present variability in Corona strains, respectively in the surface proteins S and M, leads to new Corona viruses and by this to an evading the immune system even after a first infection. These problems resulted in the failure of general vaccine development programs. On the other hand, most Corona virus strains do not cause severe diseases making vaccination programs obsolete. However, SARS, MERS and now COVID-19 are caused by highly dangerous viral strains and in case of COVID-19 are also easily transmitted.

The immune response to virus infection is characterized by three steps:
1. an initial response via the complement system (= innate immune response);
2. receptor-mediated immune responses leading to neutralizing antibody formation (IgA and IgG);
3. in most cases, a T-cell mediated response is raised eradicating infected cells;

The last two immune response steps generally also lead to memory immune cells confer life time immunogenicity.

Since normal vaccine development (attenuated or inactivated viruses) failed in SARS and MERS vaccine development, it probably will also fail in a COVID-19 immunization development.

Accordingly, it was an object of the present invention to develop a new approach for providing a vaccine, which is able to effectively immunize individuals against COVID-19 virus infection, however also provide the option to easily adapt the vaccine to other viruses and challenges, which might arise in the future.

Messenger RNA (mRNA) is the template molecule that is transcribed from cellular DNA and is translated into an amino acid sequence, i.e. a protein, at ribosomes in the cells of an organism. In order to control the expression level of the encoded proteins, mRNAs possess untranslated regions (UTRs) flanking the actual open reading frame (ORF) which contains the genetic information encoding the amino acid sequence. Such UTRs, termed the 5'-UTR and the 3'-UTR, respectively, are sections of the mRNA located before the start codon and after the stop codon. Further, mRNA contains a poly(A) tail region which is a long sequence of adenine nucleotides which promotes export of mRNA from the nucleus, translation and to some extent protects the mRNA from degradation.

Due to its chemical and biochemical properties, mRNA usually is degraded within a few minutes inside of cells, thus expression of a specific protein usually is a transient process. Moreover, the polyanionic mRNA molecule is not well suited to cross a cell membrane, which renders external delivery of mRNA extremely difficult.

Despite these challenges associated with mRNA, scientific and technological advances of the recent years have made mRNA a promising candidate for a novel class of drugs. Sahin U. et al., Nat.Publ.Gr. 13, 759-780 (2014) provide an overview on mRNA-based therapeutics and drug development. mRNA is for example used to trigger *in vivo* production of proteins like antibodies and enzymes, or to stimulate an immune response, e.g. by expressing specific epitopes or via innate immune response towards structural mRNA parts. For example, RIG-1 binds 5'-triphosphate ends of RNA and triggers a signal cascade, which results in activation of transcription factors and release of cytokines as parts of an antiviral response. Application of mRNA for stimulation of an immune response can be used in novel approaches to treat cancer, AIDS and to generate vaccines against almost any disease (cf. Pardi, N. et al., Nat.Publ.Gr. 543, 248-251 (2017) and Schlake T. et al., RNA Biol. 9, 1319-30 (2012)). Key to these exciting developments is the robust *in vitro* production of stabilized mRNA with improved translation efficiency and its delivery into cells using special transfection formulations.

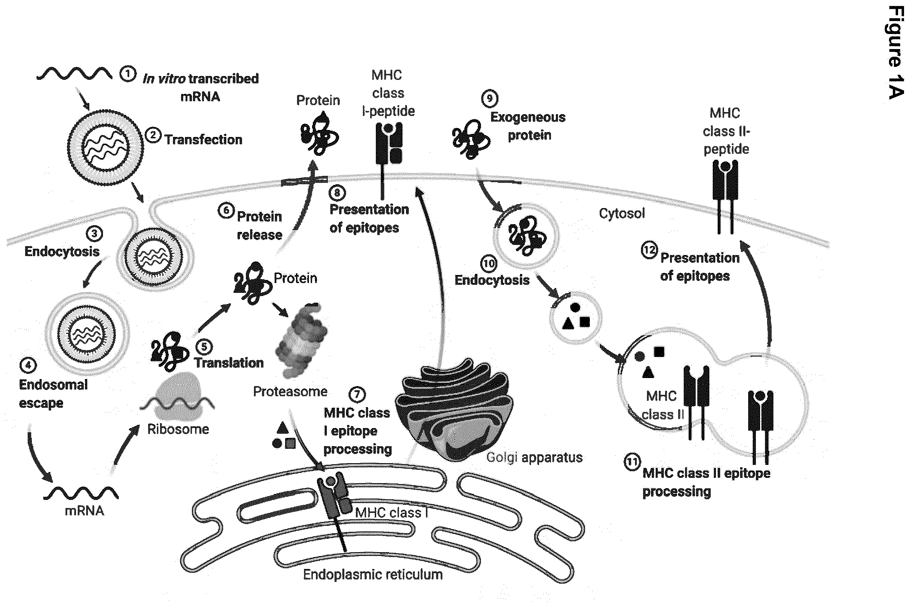

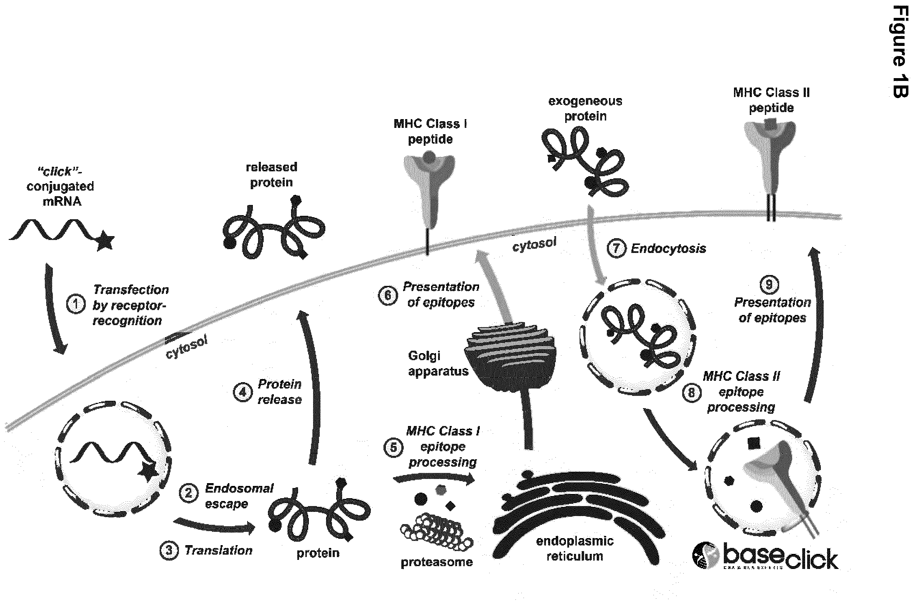

Fig. 1a) shows a depiction from A. Wadhwa et al., Pharmaceutics 2020, 12(2) and modified by baseclick GmbH to visualize the difference to LNP carrier delivery (Fig. 1b)), representing the guiding principle behind mRNA therapy which is based on providing *in vitro*-transcribed mRNA as a carrier of genetic information, thus allowing the organism to develop its own cure. In vaccination, mRNA which codes for a specific antigen is used to generate an immune response.

mRNA stability and translation efficiency depend on several factors. Especially the untranslated regions at either ends of the mRNA play a crucial role. In eukaryotic protein expression, a cap structure at the 5'-end and the poly(A) tail at the 3'-end both increase mRNA stability and enhance protein expression. In addition, the 5'-UTR contains a ribosome binding site necessary for translation and the 3'-UTR contains RNA sequences that adopt secondary structures which improve stability and influence translation. Moreover, modified natural, e.g. *N*1-methylpseudouridine, and artificial nucleotides can be incorporated to improve mRNA stability and enhance translation of the mRNA (Svitkin Y.V. et al., Nucleic Acids Research, Vol. 45, No. 10, 6023-6036 (2017)).

Delivery of mRNAs into cells can be achieved by providing mixtures containing lipids for fusion with the cellular membrane and cations to neutralize the negative charge of the oligonucleotide backbone. Special formulations have been created to optimize mRNA delivery and to confer sufficient *in vivo* stability for clinical trials. Most of the mRNA formulations, which are applied intravenously are taken up by and are expressed inside liver cells. This is due to the fact that the liver plays a major role in fatty acid metabolism and a high lipid content of the mRNA formulations therefore displays an organ-specific targeting effect. In most cases the liver is, however, not the desired target and therefore efforts are being made to modify lipid formulations to target organs, which are involved in an immune response, like e.g. the spleen (Kranz L.M. et al., Nature 534, 396-401 (2016)). Alternatively, cells of the immune system (e.g. lymphocytes) can be isolated from a patients' blood and mRNA application is performed *ex vivo* to allow targeting. Most recently, tissue-specific targeting of mRNA using antibody fragment modified lipid formulations has been disclosed (Moffett H.F. et al., Nat. Commun. 8, 389 (2017)).

Despite earlier advances and developments regarding the therapeutic applicability of mRNA either directly or indirectly, i.e. via *ex vivo* transfection of cells and returning such transfected cells to a patient, further improving inter alia the stability of mRNAs and developing new options in the context of their use as therapeutics or drugs were objects addressed by the invention disclosed in WO2019/121803, Exploring further options for exploiting the immune stimulatory effect of mRNAs in e.g. vaccine production and cancer therapy is another object of ongoing research which is addressed by the present invention.

Recent efforts of various groups and companies to develop mRNA based vaccines against the Spike protein (S) of SARS-CoV-19 rely on the delivery of the respective mRNA encapsulated in lipid nanoparticles (LNPs). However, the application of LNPs is considered problematic and also the Spike protein might not be the most effective choice of viral antigen.

### Summary of the Invention

The present invention aims at providing alternative and improved solutions to challenges caused by a pandemia as currently encountered throughout the world. The present invention relates inter alia to new mRNA based vaccines and vaccination methods which aim at providing immunity to current and future challenges by epidemic and pandemic viral outbreaks.

In a first aspect, the invention relates to a modified messenger RNA (mRNA) which encodes within its ORF an antigen such as a viral protein, an immunogenically active part of such viral protein or an anticancer protein or epitope characterized in that it contains at least one of an alkyne- or azide-modification in at least one nucleotide.

In a second aspect, the invention provides a process for the production of the inventive modified mRNA, wherein the process comprises *in vitro* transcribing mRNA from a DNA template, or alternatively performing a fermentation process using prokaryotic or eukaryotic host cells to express a DNA template contained in an expression vector wherein the process is performed in the presence of an RNA polymerase and a nucleotide mixture containing the four standard types of nucleotides required for mRNA transcription, in which nucleotide mixture at least a part of at least one of the four types of nucleotides is modified to contain an alkyne- or azide-modification.

A third aspect of the invention relates to a pharmaceutical composition, comprising a modified mRNA according to the invention as an active agent, optionally in combination with a pharmaceutically acceptable adjuvant or excipient and/or contained in pharmaceutically acceptable carrier.

A fourth aspect of the invention provides the modified mRNA according to the invention or the pharmaceutical composition for use in mRNA based therapeutic and/or prophylactic applications.

A fifth aspect of the invention relates to a kit for production and/or delivery of an inventive modified mRNA.

A sixth aspect of this invention is a method for vaccination of an individual against viral infection, especially against Corona virus infection, and most preferably against SARS-CoV-2 infection, which method comprises administering to such individual an effective amount of a modified mRNA of the present invention or of a pharmaceutical composition. A further important embodiment in the context of this sixth aspect of the invention is a method to stimulate immune response parallel to the antigen presentation in the organism. A prominent candidate for immune stimulation is the cytosolic Stimulator of interferon genes (STING) receptor. The function of the STING receptor in cells is controlled by cyclic di-nucleotides especially guanosinmonophaspate and adenosinmonophaspate (cGAMP). Drugs or chemicals such as c-di-AMP, c-diGMP, IACS-8779 and others are able to induce enhanced immune response in the organism leading to improved immune response to the antigen produced by the modified mRNA construct of the present invention.

### Detailed description of the invention and preferred embodiments

The present invention employs so-called "click chemistry" or elements thereof and applies this technique to modify mRNA molecules to impart improved stability and cell specific targeting and/or to provide for use of such modified mRNA molecules in the context of inter alia mRNA vaccine technologies, especially in combination with application of a STING agonist as adjuvant in an inventive vaccine/pharmaceutical composition.

Click chemistry is a concept which was defined in 2001/2002 by the groups of Sharpless and Meldal (Sharpless, K.B. et al., Angew. Chem. 2002, 114, 2708; Angew. Chem. Int. Ed. 2002, 41, 2596; Meldal, M. et al., J. Org. Chem. 2002, 67, 3057). Since then, especially the copper catalyzed reaction of azides with alkynes to give 1,2,3-triazoles, a variation of the 1,3-dipolar Huisgen cycloaddition (R. Huisgen, 1,3-Dipolar Cycloaddition Chemistry (Ed.: A. Padwa), Wiley, New York, 1984), has become a very widely used method to perform a click reaction. As a result of its mild conditions and high efficiency, this reaction has found a myriad of applications in biology and material sciences, such as e. g. DNA labeling for various purposes (Gramlich, P.M.A. et al., Angew. Chem. Int. Ed. 2008, 47, 8350).

In addition to the copper-catalyzed click-reaction, also copper-free, bio-orthogonal methods have been developed and all such methods can generally also be employed in the context of the present invention. E.g., strain-promoted azide-alkyne cycloaddition (SPAAC) (I.S. Marks et al., Bioconjug Chem. 2011 22(7): 1259-1263) can be used either alone or in combination with copper-catalyzed click chemistry (CuAAC) in the context of the present invention. Especially in cases in which it is desirable to perform a labelling reaction in vivo in cell culture or in a living organism, performing such reaction using SPAAC is preferable since the method does not require the use of toxic substances or external catalysts.

Click chemistry facilitates attaching reporter molecules or labels to biomolecules of interest and is a very powerful tool for identifying, locating, and characterizing such biomolecules. The method for example enables inclusion and attachment of fluorescent probes for spectrometric quantification, or of anchor molecules to allow for separation and purification of the target biomolecules. Up to date, many applications have been developed in which click chemistry is used as an underlying principle. Next-generation sequencing is one of such applications which benefits from this technique where formation of so-called "backbone mimics", i.e. non-natural alternatives for the phosphodiester bond, which can be generated by copper-catalyzed azide alkyne cycloaddition (CuACC), is used to ligate e.g. DNA fragments and adapter sequences. Despite the presence of a triazole ring instead of a phosphodiester bond, such backbone mimics are acceptable substrates for polymerase driven DNA or RNA preparation methods like PCR or reverse transcription. Detection of cell proliferation is a further field of application for click-chemistry. The methods that are normally applied include adding either BrdU or radioactive nucleoside analogs to cells during replication and detecting their incorporation into DNA. Methods involving radioactivity, however, are rather slow and not suitable for rapid high-throughput studies and are also inconvenient because of the radioactivity involved. Detecting BrdU requires an anti-BrdU antibody and applying denaturing conditions resulting in degradation of the structure of the specimen. The development of EdU-click assays has overcome such limitations by including 5-ethynyl-2'-deoxyuridine, a thymidine analog, in the DNA replication reaction. The detection via click chemistry instead of an antibody is selective, straight forward, bioorthogonal and does not require DNA denaturation for the detection of the incorporated nucleoside.

It was earlier discovered that it is possible to introduce alkyne- and/or azide-modified nucleotides during in vitro transcription of mRNA or during a fermentation process for producing mRNA to result in a correspondingly modified mRNA, cf. WO2019/121803. The alkyne- or azide-modification can be included in only some or all elements contained in the mRNA, and needs to be included in at least one of the UTRs, ORF and poly(A) tail. The 5'cap structure preferably does not contain such alkyne- or azide-modifications, as changes to the cap structure can interfere with efficient binding of initiation factors like eIF4E, eIF4F and eIF4G and thus drastically decrease translation efficiency. The presence of such modification on the one hand stabilizes the mRNA and on the other hand provides specific anchor sites for attachment of tissue- or cell-specific ligands or targeting molecules via click chemistry. Thus, the modification of mRNA not only provides for a higher stability of the mRNA molecules, but also provides new options for targeted delivery of mRNAs to specific organs or cell types in the context of therapeutic or prophylactic applications, especially immunization/vaccination. A correspondingly modified mRNA is a first subject matter of the present invention, which encodes within its ORF an antigen such as a viral protein, an immunogenically active part of such viral protein or an anticancer protein or epitope and which contains at least one of an alkyne- or azide-modification in at least one nucleotide.

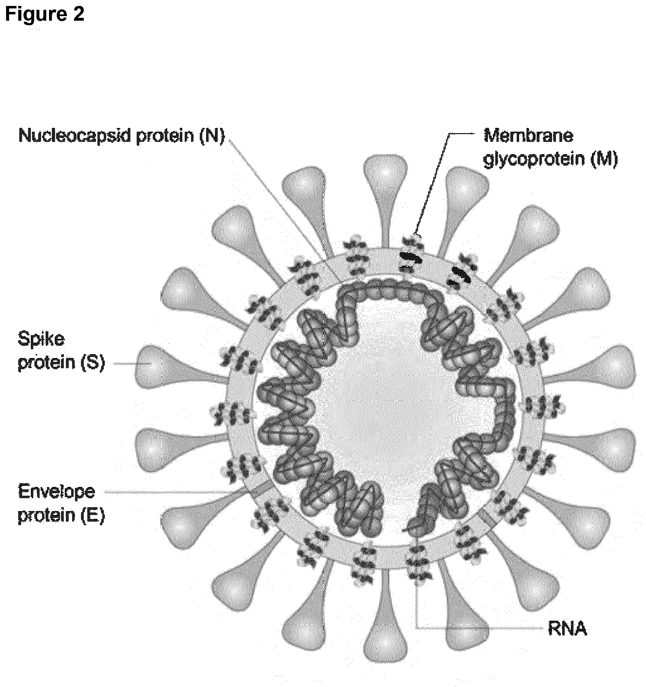

According to a preferred embodiment of the present invention, the mRNA encodes a Corona virus protein, preferably a Corona virus (nucleo)protein and, most preferably, at least one of nucleoprotein N and envelope protein E of SARS-CoV-2, or an immunogenically active part thereof. Fig. 2 shows a SARS-CoV-2 virus particle and points out relevant constituents.

Within the context of the present invention, the term "immunogenically active" is intended to include parts of a protein or epitopes, which give rise to a primary immune response and also provide for immunological memory which triggers subsequent immune responses to the same antigen.

Depending on which type of nucleotide or nucleotides are included in alkyne-or azide-modified form during in vitro transcription or during mRNA production in prokaryotes or eukaryotes via fermentation, the resulting modified mRNA can contain modifications not only in the 5'- and 3'-UTRs and the ORF, but also in the poly(A) tail region. As apparent to the skilled person, including e.g. one or more of modified CTP, GTP and UTP leads to a modification within the UTRs and the ORF, while additionally including modified ATP results in a modification also of the poly(A) tail region. Including only alkyne- and / or azide-modified ATP during the transcription leads to modifications in the UTRs, the ORF and the poly(A) tail region.

No severe negative effects caused by the presence of alkyne- or azide-modified nucleotides within the mRNA of the invention have been observed. Depending on the amount of modified nucleotides included in the reaction, the in vitro and in vivo transcription efficiency can be as effective as in cases where only non-modified nucleotides are present in the reaction mixture, or slightly decreased. Furthermore, the modification of the mRNA does not seem to impair translation of mRNA during protein production at the ribosomes. Depending on the circumstances, the amounts of modified nucleotides to be included in the in vitro transcription reaction or the fermentation process can be adjusted to either provide maximum mRNA yield or maximum modification. For instance, in order to target specific cells and cell receptors, it might be sufficient to include only one or a few respective ligand molecules to achieve the desired effect, whereas for maximum efficiency it is also possible to include a higher number of ligand molecules.

As will be explained later in more detail, including alkyne- or azide-modified nucleotides has a stabilizing effect on mRNA. It is to be expected that the stabilizing effect of the inventive modification is most pronounced if such modification is distributed over the complete mRNA molecule. In such case, subsequent attachment of functional molecules via click chemistry will occur also uniformly over the whole mRNA molecule and can even provide for an enhanced stabilizing effect.

However, in the context of the invention and the intended immunization of individuals by efficiently expressing the antigen within targeted cells, it may be important to restrict the inclusion of such functional molecules to a part of the mRNA molecule, which is not involved in subsequent translation of mRNA during protein expression. For such purpose, it can be desirable to include modified nucleotides in the poly(A) tail region only whereby it can be ensured that ribosomal activity is not impaired by the presence of especially longer or bulkier labels or functional molecules like ligands or targeting molecules.

The present invention therefore also provides a modified mRNA containing alkyne- or azide-modification in the poly(A) tail region only. Instead of including alkyne- or azide-modified nucleotides during in vitro transcription of a DNA template or a fermentation process, a modification in only the poly(A) tail region can be achieved for any desired mRNA by performing an addition reaction in the presence of poly(A) polymerase and alkyne- or azide-modified ATP.

By controlling the amount and type of alkyne- or azide-modification in the modified mRNA of the invention it is possible to conveniently and easily adapt the resulting mRNA to impart stabilization and options for post-enzymatic attachment of molecules of interest as required and viable with regard to any intended application.

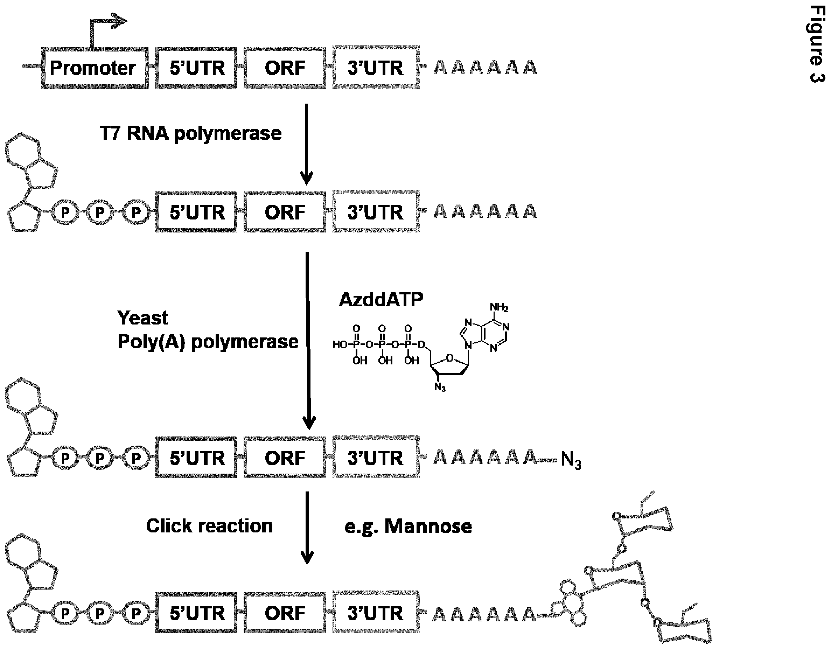

Within the context of the present invention, the azide- or alkyne-modification can be included at the nucleobase or at the 2'-position of the ribose unit of the respective nucleotide. In very special cases, inclusion of a nucleotide containing the modification at the 3'-position of the ribose is also possible. In such case, the enzymatic poly(A) addition reaction is terminated upon inclusion of one modified nucleotide. Fig. 3 shows a preferred embodiment of the present invention in which a ribonucleotide which carries a modification at the 3' position of the ribose is added during the poly(A) polymerase reaction. In such case, an mRNA molecule is obtained which carries the modification at the 3'-end of the molecule which can then be modified to include a functional molecule or ligand via click reaction.

In another preferred embodiment of this aspect of the present invention, the modified mRNA contains an alkyne- and/or an azide-modification at the nucleobase or the 2'-ribose position in at least one of the nucleotides within at least one of the UTRs, the ORF and optionally also the poly(A) tail region, and additionally a chain-terminating alkyne- or azide-modification at the 3'-position of the ribose in the poly(A) tail.

In a different preferred embodiment, the mRNA of the present invention does not contain a chain-terminating alkyne- or azide-modification at the 3'-ribose position in the poly(A) tail region.

The modified nucleotide included in the mRNA of the present invention can be derived from a natural nucleotide and especially one of the standard nucleotides with adenine, cytosine, guanine or uracil bases, or it can be a modification of another naturally occurring nucleotide (e.g. pseudouridine derivative) or even a non-naturally occurring molecule (e.g. F. Eggert, S. Kath-Schorr, Chem. Commun., 2016, 52, 7284-7287) which does not negatively affect transcription and/or translation and the function of the resulting modified mRNA. Preferably the modified nucleotide is derived from a natural nucleotide or a naturally occurring nucleotide within mRNA.

Suitable alkyne- and azide-groups for click reactions are known and available to the skilled person and all such groups can be used to prepare modified nucleotides and modified mRNAs in the context of the present invention. The alkyne-modified nucleotide preferably is an ethynyl-modified nucleotide, more preferably 5-ethynyluridine phosphate or 7-ethynyl-7-deazaadenine phosphate. While it is in principle also possible to employ higher alkyne-modified nucleotides, especially propynyl or butynyl modified nucleotides and even C-C triple bond-containing ring systems, possible negative effects on e.g. the transcription or poly(A) polymerase reaction efficiency as well as on a further translation of the mRNA into a protein will have to be considered when selecting suitable alkyne molecules. Azido-modifications for nucleotides which are useful in the present invention can, e.g., also include azidoalkyl groups in which the alkyl part preferably is a lower alkyl group, especially a methyl, ethyl or propyl group. As an azide-modified nucleotide, preferably 5-(3-azidopropyl)-uridine phosphate or 8-azidoadenine phosphate are considered for inclusion in the inventive mRNA. An example for an azide-modified nucleotide causing termination of the poly(A) addition reaction is 3'-azido-2',3'-dideoxy adenine phosphate.

In principle, all nucleotides of the at least one type of modified nucleotide can be alkyne-or azide-modified, or alternatively, only a part of such nucleotides is present in modified form. In a preferred embodiment of the present invention and depending on the desired modification and modification rate, ratios of modified versus non-modified forms of the various nucleotides can range from 1:100 to 10:1, preferably 1:10 to 10:1, further preferably 1:4 to 4:1, and also preferably 1:2 to 2:1. Preferably, a 1:1, 1:4 or 1:10 combination of modified to non-modified nucleotide is included in the mRNA of the invention.

As mentioned above, the presence of alkyne-or azide-modified nucleotides or nucleobases in the modified mRNAs of the present invention confers a stabilizing effect. On the one hand, the attack of endoribonucleases is restricted to some extent by an internal modification. Extension of the poly(A) tail region during poly(A) polymerase based addition of modified ATPs at the 3'-end leads to a further stabilizing effect. Attack on and degradation of mRNA molecules by exoribonucleases occurs at the two ends of RNA. The mRNA according to the invention contains a cap at the 5'-end which provides protection from degradation at that side. Including additional modified adenosine nucleotides at the 3'-end imparts further protection as the attack of exoribonucleases in 3'→5' direction is impeded and degradation reaching the core mRNA, especially the ORF, is delayed.

In a preferred embodiment of the present invention, functional molecules can be introduced into the modified mRNA via click reaction with a correspondingly modified alkyne- or azide-containing functional molecule. As for the nucleotide modification, also for the modification of functional groups, suitable alkyne- and azide groups are known to the skilled persons and the preferred examples for such groups are applicable as described above. The reaction of an alkyne-modified nucleotide within the modified mRNA and an azide-containing functional molecule or the reaction of an azide-modified nucleotide within the modified mRNA of the invention and an alkyne-containing functional molecule is performed under conditions to conduct the click reaction and leads to formation of the 5-membered heterocyclic 1,2,3-triazole moiety which forms the link between mRNA and functional molecule. According to the present invention, the term alkyne-containing functional molecule also encompasses C-C triple bond-containing ring systems like cyclooctynes which have been considered especially in the context of SPAAC reactions and in vivo labelling via bio-orthogonal ligation reactions.

The type and size of functional molecules are determined by the intended use. Functional molecules to be included in the modified mRNA via a click reaction are not restricted and are preferably cell- or tissue-specific ligands that mediate targeted uptake of the mRNA into specific tissues or cells including cancer tissues and cells or target lymphocytes, in particular MCH1 peptide presenting cells, such as mast cells, The functional molecules allow to attach or anchor the mRNA at the cell-surface. Such cell- or tissue-specific targeting can be achieved for example by using specific antibodies or antibody fragments, peptides, sugar moieties, small molecules (e.g. folic acid) or fatty acid moieties as the cell- or tissue-specific ligands. Respective substances have been described for a large number of targeting applications and are available to the skilled person. Some preferred and exemplary targeting molecules are antibodies or antibody fragments, especially anti-CD20 or anti-CD19 antibodies or fragments thereof, or receptor ligands which target cell specific receptors like e.g. the epidermal growth factor receptor, folate which targets the folate receptor, apolipoproteins which target endogenous low-density lipoprotein receptors or arachidonic acid which targets the endogenous cannabinoid receptors. Also, the amino acid sequence RGD or similar sequences have been found to mediate cell adhesion and can also be considered as preferred ligands within the context of the present invention.

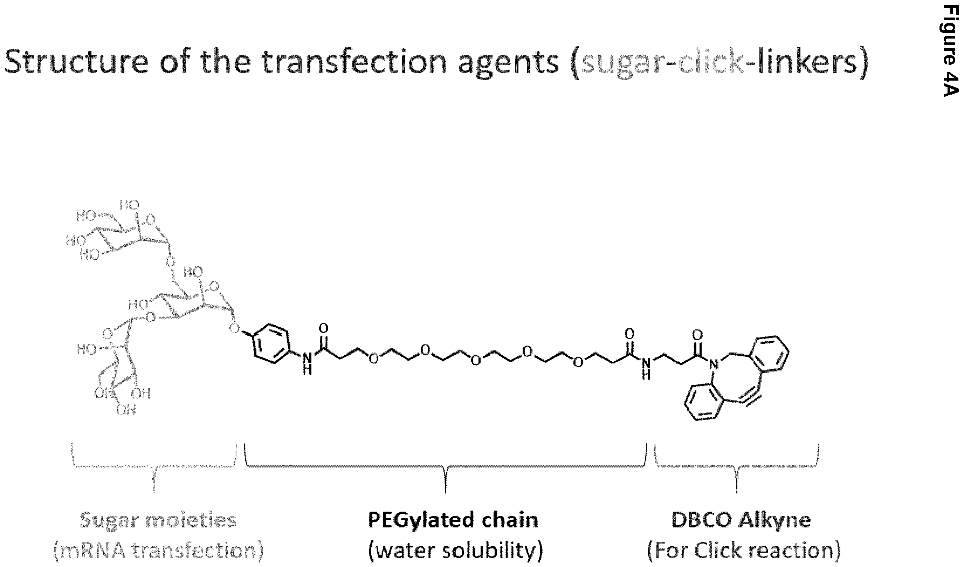

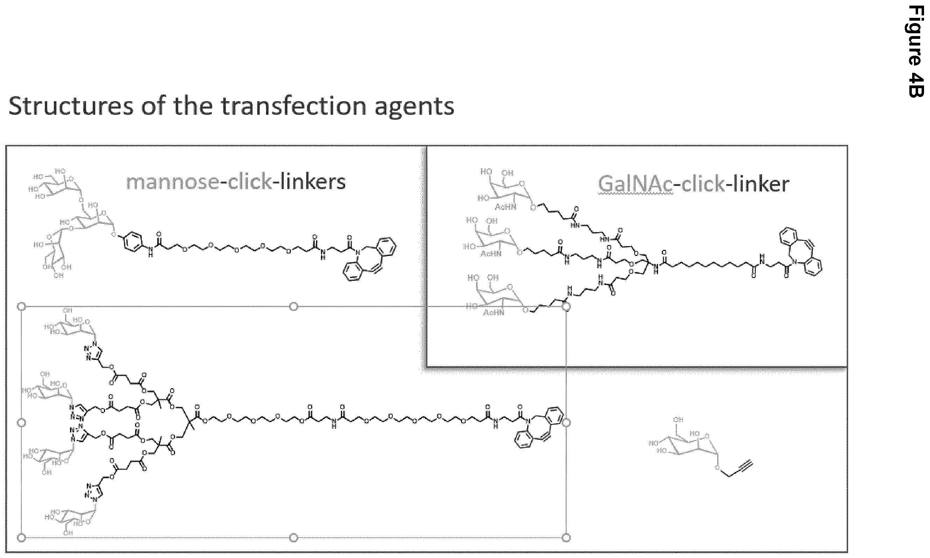

Further preferred functional molecules or cell- or tissue-specific ligands include sugar moieties as shown in Fig. 4. The ligand molecules preferably include one or more sugar moieties, which are linked to an alkyne or azide click reaction moiety via a spacer. Such spacer can be straight-chained or branched and, in both cases, one or several sugar moieties can be attached to the one or more chain termini. In preferred embodiments, the sugar moiety is selected from aldoses, especially glucose, galactose and mannose; ketoses, especially fructose; and sugar derivates, especially N-acetyl galactosamine (GalNAc). Such ligands can act as transfecting agents since they facilitate targeting and transfection of certain kinds of cells, especially cells of the immune system. Sugar moieties, especially mannose and GalNAc, allow for receptor recognition by C-type lectins.

Processes for producing such functional moieties, which include a sugar moiety attached to an azide or alkyne click moiety via a linker are exemplarily depicted in Figs. 5 and 6. However, other sugar moieties as well as different spacer molecules and alkyne- or azide moieties can be included. Development of an mRNA vaccine using such ligand is schematically represented in Fig. 7.
The presence of functional molecules attached to the mRNA can further increase mRNA stability against nuclease degradation and it has been shown that partial as well as full replacement of at least one of the natural nucleotides within the mRNA for an alkyne- or azide-modified analogue and even attachment of functional molecules thereto does not hamper translation of the mRNA molecule.

In addition to including either alkyne-modified or azide-modified nucleotides, it is also possible that an inventive modified mRNA contains at least one nucleotide in partially or completely alkyne-modified form and at least one other nucleotide in partially or completely azide-modified form. A further option is an mRNA including at least one type of nucleotide in partially or completely alkyne- as well as in partially or completely azide-modified form. Such mRNA contains two different anchor modifications to which different functional molecules can be attached in a downstream post-enzymatic click reaction. For example, but without limiting to such specific embodiment, a first alkyne-modified cell-specific targeting group as well as a second different azide-modified targeting group can be attached resulting in another preferred embodiment of a modified mRNA according to the present invention which provides for maximum specificity and/or effectivity of cell targeting and mRNA uptake into the cells.

It is also possible and preferred within the context of the invention to provide a modified mRNA containing at least an azide-modified nucleotide and an alkyne-modified nucleotide, wherein e.g. at the azide-modified nucleotide a functional molecule has been attached via a biorthogonal reaction, e.g. SPAAC in vitro, whereas the alkyne-modified nucleotide is available for a downstream in vitro labelling reaction via a CuAAC reaction. If CuAAC reaction conditions are applied to the double labeled mRNA (containing alkyne and azide functions), it is possible to circularize the mRNA, which is, e.g., a valuable alternative to using self-splicing introns (DOI: 10.1038/s41467-018-05096-6).

It is for example also conceivable for the inventive modified mRNA to contain one kind of modification in the UTRs and the ORF and another modification solely in the poly(A) tail. Such modification can be effected by performing first a transcription reaction to introduce one or more first types of modified nucleotide and then following up with a poly(A) polymerase reaction using a second type of modification-containing ATP.

It will be apparent to the skilled person that numerous modifications and combinations of modifications are possible in the context of the present invention. Further, it is also possible to include different functional groups based on the presence of the alkyne- and/or azide-modifications on the mRNA molecule, but rather also by consecutive addition under click reaction conditions of different appropriately modified functional molecules. Consequently, the present invention provides a vast number of options and a convenient modularity in order to adapt the modified mRNA to the intended use in an optimal manner.

Apart from including alkyne- and / or azide-modified nucleotides, the present invention generally also allows for other modifications in the nucleotides as far as such other modifications do not adversely affect mRNA production or the intended use of the resulting mRNA to an extent which is not acceptable when contemplating the intended use (i.e. the modification is compatible with the modified mRNA within the context of the invention). As an example of such other modified nucleotide or nucleotide derivative that can be included in the mRNA, pseudouridine-5'-triphosphate (pseudo-UTP) can be considered. Pseudouridine (or 5'-ribosyluracil) was the first modified ribonucleoside that was discovered. It is the most abundant natural modified RNA base and is often designated as the "fifth nucleoside" in RNA. It can be found in structural RNAs, such as transfer, ribosomal and small nuclear RNA. Pseudouridine has been shown to enhance base stacking and translation. Further, pseudouridine-5'-triphosphate is able to impart advantageous mRNA characteristics such as increased nuclease stability and altered interaction of innate immune receptors with in vitro transcribed RNA. Incorporation of pseudo-UTP and also further modified nucleotides, like N1-methylpseudouridine and 5-methylcytidine-5'-triphosphate into mRNA, have been shown to decrease innate immune activation in culture and in vivo while simultaneously enhancing translation (B. Li et al., Bioconjugate Chemistry, 2016, 27, 849-853 and Y. Svitkin et al., Nucleic Acid Research, 2017, 45, 6023-6036). Inclusion of these and other suitable and compatible nucleotides, nucleotide analogues or non-naturally occurring molecules as described earlier in this specification, in alkyne- or azide- modified or in non-modified form is therefore a further option and preferred embodiment of the present invention.

Within the context of the present invention, the modified mRNA of the present invention can be used together with substances which are required or preferably present for a certain application. For example, for in vivo administration as a vaccine, substances which facilitate mRNA uptake by cells are preferably combined with the mRNA. Lipid formulations as well as nanocarriers (e.g. as described by Moffett et al., mentioned supra) can be included in respective compositions and formulations within the context of the present invention. Accordingly, a mixture of substances containing the modified mRNA and at least one other substance as mentioned above, or a kit of parts in which the modified mRNA and at least one other suitable substance are provided in different containers for subsequent combined use are further subjects of the present invention.

WO2010/037408 describes an immunostimulatory composition comprising an adjuvant component comprising at least one mRNA preferably complexed with a cationic or polycationic compound, and at least one free (i.e. non-complexed) mRNA which encodes at least one therapeutically active protein, antigen, allergen and/or antibody.

In this context, also a combination of a modified RNA of the present invention with a further modified mRNA acting as an adjuvant can be applied. Also, for such uses, it can not only be taken advantage of the possibility of specific targeting and delivery to cells provided by the present invention but also of the stabilization conferred to the (m)RNAs by the modification as disclosed earlier herein.

Another subject of the present invention is a process for producing the modified mRNA of the present invention. According to a first process, mRNA is transcribed in vitro from a DNA template in the presence of an RNA polymerase, usually T3, T7 or SP6 RNA polymerase, and a nucleotide mixture containing at least the four standard types of nucleotides (ATP, CTP, GTP, UTP) required for mRNA transcription and optionally naturally occurring modified nucleotides, like e.g. N1-methylpseudouridine triphosphate, or even suitable artificial nucleotides. In addition, to improve the translation efficiency it is important to generate a 5'-cap structure, e.g. 7-methylguanylate for eukaryotes. At least a part of at least one of the standard nucleotides, naturally occurring modified nucleotide analogue or suitable artificial nucleotide analogues is modified to contain an alkyne-or azide-modification at the nucleotide.

Depending on which type of nucleotide is used for the process, the modification will be effected in the UTRs and the ORF only (for modified CTP, GTP or UTP, or their analogues) or in all of the UTRs, the ORF and the poly(A) tail (for modified ATP alone or in combination with one or more of modified CTP, GTP or UTP, or their analogues).

The conditions and methods to perform in vitro mRNA transcription (IVT) as well as a poly(A) polymerase addition reaction are well known to the skilled person (e.g. Cao, G.J et al, N. Proc. Natl. Acad. Sci. USA. 1992, 89, 10380-10384 and Krieg, P. A. et al,. Nucl. Acids Res. 1984, 12, 7057-7070)

Such conditions and methods are not particularly critical as long as a satisfactory yield of modified mRNA is obtained. In this context, also the kind of DNA template used within the first described process is not particularly critical. Usually, DNA to be transcribed is included in a suitable plasmid, however it can also be used in linear form. Additionally, a DNA template usually contains a promoter sequence, especially a T3, T7 or SP6 promoter sequence.

During the process of producing the modified mRNA of the present invention, the obtained mRNA is preferably capped using well-known methods (Muthukrishnan, S., et al, Nature 1975, 255, 33-37). Required reactants for the capping are commercially available, for example A.R.C.A. (P1-(5'-(3'-O-methyl)-7-methyl-guanosyl) P3-(5'-guanosyl)) triphosphate, a cap analog) (Peng, Z.-H. et al, Org. Lett. 2002, 4(2), 161-164). Preferably, as an alkyne-modified nucleotide, ethynyl-modified nucleotides, most preferably 5-ethynyl UTP or 7-ethynyl-7-deaza ATP, are included in the process. As an azide-modified nucleotide, preferably 5-(3-azidopropyl) UTP, 3'-azido-2',3'-dideoxy ATP(at the 3'-end only) or 8-azido ATP is used.

Within the context of the present invention, it is preferred to perform the transcription process using T7 RNA polymerase and to provide the DNA template in a suitable vector for efficient template production using microorganisms and subsequent in vitro transcription after linearization of the vector.

As an alternative to in vitro transcription, also a fermentation process in prokaryotic or eukaryotic systems for producing the mRNA of the invention is included in the context of the present invention. For this purpose, a DNA template, which is usually included in a suitable expression vector, preferably a plasmid containing the DNA of interest under control of an RNA polymerase promoter, is introduced into host cells or microorganisms and respective nucleosides or nucleotide prodrugs (to allow sufficient cellular uptake) as described above are included in the culture medium. Fermentative RNA production is known to the skilled person, cf. e.g. Hungaro et al. (J Food Sci Technol. 2013 Oct; 50(5): 958-964).

For illustration purposes, however not to restrict to such specific process, the production of alkyne-, azide- and click-modified mRNA via fermentation is described in more detail for a bacterial system: A DNA template, encoding the mRNA of interest under control of an RNA polymerase promotor, is introduced into bacterial cells. Preferably this is done via transfection of a plasmid. The design of the sequence is important and preferably contains all of several elements necessary for production of the desired mRNA: RNA polymerase promotor (e.g. T7 or SP6 promoter); the open reading frame of interest (ORF); and preferably also a sequence encoding the poly(A) region (preferably 100-120 nt long). Moreover, the plasmid contains an origin of replication and a selection marker for controlled growth and amplification in cell culture. It is preferable to have a gene regulatory element for the open reading frame, e.g. a lac operon, to selectively induce expression of the mRNA upon addition of an external compound. Important is the poly(A) region, necessary for discrimination of the mRNA from all the other RNAs (e.g. bacterial mRNAs, tRNAs and rRNAs) during purification and to provide the mRNA product with sufficient stability and translation efficiency. A poly(A) tail region can, however, also be introduced or a comparatively short poly(A) tail can be extended and possibly also modified via polymerase A addition reactions as described within the context of this invention after the fermentative production of the mRNA.

Alkyne- or azide-modified nucleosides are added to the growing medium and are taken up by the bacterial cells via transporters or passive mechanism (J. Ye, B. van den Berg, EMBO journal, 2004, 23, 3187-3195). Intracellularly these nucleosides are phosphorylated by kinases to the corresponding triphosphates and can be incorporated into the mRNA. Since the monophosphorylation of the nucleosides is a slow process, it is possible to feed monophosphate prodrugs of the nucleosides to increase intracellular nucleotide concentrations (like for sofosbuvir).

In case of azide-modified mRNA, a click-reaction using biorthogonal chemistry, e.g. strain promoted azide-alkyne cycloadditions (SPAAC) can be performed in cell culture. Therefore, preferably cyclooctyne modified tags/labels or functional molecules are added to the medium.

The newly synthetized mRNA, which includes the modified nucleosides within the sequence, is then e.g. purified by the usage of poly(T) oligonucleotides attached to a specific resin and/or beads. e.g. of the mRNA isolation kit form Sigma Aldrich (cat No: 000000011741985001).

It is well known that the mRNA of prokaryotic cells does not contain a poly(A) region or when it does, it is not longer than 20nt, which is not enough to be taken up by the poly(T) oligonucleotides attached to the resin and/or beads, thus allowing for an efficient separation of the desired mRNA form prokaryotic mRNA. Thus, a fermentatively produced mRNA without a poly(A) tail regions or without a sufficiently long poly(a) tail region needs to be purified by other known methods via chloroform phenol extraction, precipitation and subsequent purification of the crude cellular RNA by ion exchange chromatography.

The bacterial strain, e.g. E.coli BL21(DE), needs to have the RNA polymerase, e.g. the T7 RNA polymerase, integrated in the genomic DNA (e.g. DE3 strains). Production of the mRNA is then possible when a plasmid containing the T7 promotor is transformed and can introduce the alkyne- or azide-modified nucleoside during in vivo transcription within the bacterial cell.

It is also well known that the prokaryotic mRNA is lacking the 5'CAP structure. This important element of the inventive modified mRNA can be introduced after the purification of the mRNA or it can be introduced concurrently by co-transforming the bacterial cell with another plasmid expressing the eukaryotic capping enzyme.

As a further alternative, it is possible to produce the modified mRNA of the present invention via solid phase or phosphoramidite synthesis and include modified nucleotides as described above. Especially in cases where the (m)RNA is intended for use as an adjuvant and shorter molecules or non-coding sequences can be considered for such purpose, synthetic preparation can be convenient and effective. Respective methods are available to the skilled person and described e.g. in Marshall, W. S. et al. Curr. Opin. Chem. Biol. 2004, Vol. 8, No. 3, 222-229.

A second process within the context of the present invention allows for modification of the poly(A) tail region only, by first providing an mRNA of interest by any suitable method and adding modified alkyne- or azide-modified ATP (or analog) in a poly(A) polymerase addition reaction. Such poly(A) polymerase addition reactions and suitable conditions are well-known to the skilled person and respective reaction kits are commercially available.

While the first and the second process described above can be used separately to provide modified mRNA of the present invention, it is also possible to use a combination of in vitro transcription or synthetic mRNA production and poly(A) polymerase addition reaction to include modified alkyne and/or azide-modified nucleotides in the UTRs, the ORF and the poly(A) tail during the mRNA transcription step. By additionally performing the second process, i.e. a poly(A) polymerase addition reaction, a further extension of the poly(A) tail can be achieved, wherein ATP is at least partly included in an alkyne- or azide-modified form which optionally is different from the modification that is introduced by the first process.

In case of a fermentative production of an mRNA in prokaryotes with or without a poly(A) tail region it is also possible to include a poly(A) polymerase addition reaction in order to provide such poly(A) tail or to extend an existing poly(A) tail region. In such embodiment, including modified adenine nucleosides or adenine nucleotide prodrugs for the reaction in the feeding medium is a preferred option. Alternatively, modified nucleoside triphosphates for the mRNA fermentation process can be internalized directly using either expression of nucleotide transporter proteins (D. A. Malyshev, K. Dhami, T. Lavergne, T. Chen, N. Dai, J. M. Foster, I. R. Correa, Jr., F. E. Romesberg, Nature 2014, 509, 385-388.) or by adding artificial molecular transporters in the feeding medium (Zbigniew Zawada et al., Angew. Chem. Int. Ed. 2018, 57, 9891 -9895).

The processes for producing the mRNA of the invention can be performed using only one type of modified nucleotide or including one or more nucleotides comprising desired alkyne- or azide-modification. Within the context of the present invention, it is preferred to include one or two types of equally modified nucleotide, most preferably alkyne- or azide-modified uracil or adenine. As far as the alkyne-modification is concerned, it is most preferable to include an ethynyl group which, due to its size, is least prone to negatively affect the transcription reaction.

In another preferred embodiment of the invention, two differently modified nucleotides are included with the nucleotide mixture during transcription. Such a process results in a modified mRNA molecule which contains an alkyne- as well as an azide-modification.

No particular restrictions have been observed concerning the amount of modified nucleotides to be included during transcription or fermentation or via poly(A) polymerase reaction. Theoretically, all nucleotides employed in the in vitro transcription can be modified to contain alkyne- or azide-modified nucleobases. It is, however, preferred to use one or two types of modified nucleotides and also to include such nucleotides in modified as well as in non-modified form. Depending on the desired modification rate, it is preferred to include modified versus non-modified forms of the various nucleotides in a ratio of 1:100 to 10:1, preferably 1:10 to 10:1 and further preferably 1:4 to 4:1 or 1:2 to 2:1. Most preferably, only one type of modified nucleotide is employed which can be present in modified form only, or in combination with the non-modified form in the above-mentioned ratios. Preferably, a 1:1, 1:2 or 1:10 combination of modified to non-modified nucleotide is provided.

The ratios for introduction of modified nucleotides correspond with the number of modifications present in the inventive mRNA. Accordingly, the ratio of modified to non-modified nucleosides within the mRNA or the various parts, i.e. the UTRs and the ORF, or the UTRs, the ORF and the poly(A)tail, or the poly(A) tail alone is also preferably 1:100 to 10:1, more preferably 1:10 to 10:1 and further preferably 1:4 to 4:1 or 1:2 or 2:1, as well as most preferably 1:1, 1:2 or 1:10.

It is further possible and can be desirable to include differently modified natural nucleotides, e.g. pseudouridine or N1-methyl-pseudouridine and/or artificial nucleotides or nucleotide derivatives to improve mRNA stability and enhance translation of the produced mRNA. More information with regard to differently modified nucleotides and their incorporation into mRNA during in vitro transcription can be derived from Svitkin, Y.V. et al., Nucleic Acids Research 2017, Vol. 45, No. 10, 6023-6036.

Modified mRNA of the invention which is produced by in vitro mRNA transcription, by poly(A) polymerase addition reaction on an existing mRNA of interest, by a fermentation process or even completely synthetically and which comprises at least one of an alkyne- or azide-modification can further be modified via a click reaction to incorporate other molecules of interest, especially functional molecules as already explained above. It is especially preferred to incorporate a cell-specific targeting group or ligand, which target lymphocytes, in particular MHC1 peptide presenting cells, such as mast cells. Such functional molecules or ligands have been described in more detail above and can be e.g. sugar moieties, fatty acid moieties, cell-type specific antibodies or fragments thereof, like anti-CD20 or anti-CD19 antibodies or fragments thereof.

Selective modification of solely the poly(A) tail region can be achieved as described above, when poly(A) polymerase adds azide- or alkyne-modified ATP or ATP derivatives to the mRNA. Subsequent click labeling of this modified poly(A) tail has only minor effects on translation (as the sequence is not translated) and can be used, e.g. for tissue-specific ligands that mediate targeted uptake of the mRNA or to increase mRNA stability against nuclease degradation, as explained above. Especially in cases in which it is desired to attach very large molecules or molecules that due to other reasons impair translation, coupling via the poly(A) tail can be a preferred or even a mandatory approach.

The click reaction is well known to the skilled person and it is generally referred to Sharpless et al. and Meldal et al., mentioned supra. The overall conditions for the click reaction are described in these documents and it is further referred to disclosure in Himo F. et al., J. Am. Chem. Soc., 2005, 127, 210-216, which relates to the preferred copper-catalyzed azide-alkyne cycloaddition (CuAAC). It is also referred to EP 2 416 878 B1 with regard to the conditions and reactants for the click reaction as well as to EP 17 194 093, wherein a preferred method for coupling a first molecule to a second molecule in a click ligation reaction is described. In this context, the copper-catalyzed click reaction is preferably performed in the presence of divalent metal cations in the reaction mixture, most preferably in the presence of Mg²⁺.

While the above-mentioned documents describe click reactions in the context of ligating DNA molecules, in general, the same conditions can be applied within the context of the present invention. Thus, the click reaction is preferably carried out in the presence of a heterogeneous Cu (I) catalyst. Further, it is preferred to include a Cu (I) stabilizing ligand and/or organic solvents, especially DMSO to improve the efficiency of the click reaction, and/or divalent cations (e.g. as disclosed in PCT/EP2018/076495).

In a further preferred embodiment, the click reaction is performed as a strain-promoted azide-alkyne cycloaddition reaction (SPAAC) as described earlier with regard to the modified mRNA of the invention. The exact conditions of a CuAAC or a SPAAC reaction can be adapted to the individual circumstances as long as the basic requirements that are known to the skilled person are observed. As mentioned above, SPAAC can also be performed inside of cells. Introducing an alkyne- or azide-modified label into such cells can be useful in order to, after transfection of a modified mRNA into cells, monitor e.g. the location of the mRNA in the cell.

The present invention allows to produce in a modular and highly efficient manner modified mRNA molecules which contain modifications which impart a stabilizing effect on the mRNA. The modifications are also useful as anchor molecules to which other substances and molecules can be linked via a click reaction. Such click reaction is preferably performed downstream and separately from the transcription reaction which is a tremendous advantage, especially where large and bulky molecules of interest are to be ligated to the mRNA, which would completely disrupt the transcription reaction.

Thus, the inventive mRNAs and the processes for their production for the first time provide an easy and reliable method to produce stabilized and customarily modified mRNAs which can be used to provide improved cell- or tissue-specific targeting and delivery for specific uses in therapy or vaccine preparation.

The modified mRNA of the present invention can be applied directly to an individual. A further subject-matter of the present invention therefore is a pharmaceutical composition which comprises a modified mRNA of the present invention as an active agent. As already mentioned, mRNA based therapeutics have recently become important research subjects. A large number of applications for mRNA as therapeutic agents has been described (e.g. Sahin et al, Schlake et al. and Kranz et al, all mentioned supra) and the modified mRNA of the present invention can not only be used in all such applications but can even provide for advantages and improvements thereto. Based on the enhanced stability of the modified mRNA and further based on a preferably present targeting group or tissue- or cell specific ligand, various problems can be solved. An enhanced stability accounts for e.g. a prolonged translation into protein compared to a non-modified mRNA. Further, the presence of a tissue- or cell-targeting group allows for targeted administration and high specificity of a therapeutic or immunogenic treatment.

The pharmaceutical composition according to the invention is preferably applied as mRNA vaccine. Vaccination is effected based on in situ protein expression to induce an immune response. Since any protein can be expressed from the modified mRNA of the present invention, the present pharmaceutical compositions can offer maximum flexibility as regards the desired immune response. Using the modified mRNA also provides a very fast immunization alternative compared to conventional methods for which it is necessary to produce various protein constituents or even inactivated viral particles. Conventional methods usually require performing different production processes whereas using the present invention, various mRNAs encoding different proteins or protein parts relating to the infectious agent can be produced in the same preparation process. Immunization by mRNA vaccination can even be achieved by single vaccinations and using only low mRNA doses. As opposed to DNA vaccines, RNA vaccines do not need to cross the nuclear envelope, but it rather is sufficient for them to reach the cell cytoplasm by crossing the plasma membrane. Further information regarding the development of mRNA vaccines is disclosed e.g. in Schlake et al., mentioned supra, and is applicable also in the context of the present invention. The pharmaceutical composition including the modified mRNA of the invention can be employed as prophylactic as well as therapeutic vaccines. The vaccines can be directed against any kind of pathogens, e.g. viruses like Zika virus which recently has become a major focus of attention (Pardi et al, mentioned supra), and, even more relevant at present, against RNA viruses which cause severe respiratory diseases, e.g. Corona viruses and especially SARS-CoV-2.

In addition to a vaccination against exogenous pathogens, the pharmaceutical compositions of the present invention can also be used as anti-tumor vaccines or to stimulate the immune system within the context of cancer immunotherapy. Especially systemic RNA delivery to dendritic cells or macrophages offers the possibility to exploit antiviral defense mechanisms for cancer immunotherapy as described by Kranz et al., mentioned supra. Targeting e.g. macrophages or dendritic cells with an mRNA expressing a protein specific to or within the context of a certain type of cancer leads to presentation of parts of such protein by MHC molecules and elicits a potent and specific immune response. Accordingly, the modified mRNA of the present invention can also be used in the context of antigen-encoding mRNA pharmacology.

In the context of RNA-based immunotherapy and vaccination, it is especially preferred to include an (m)RNA adjuvant as described above in an immunostimulatory pharmaceutical composition. The adjuvant provides for a stimulation of the innate immune response and thus further enhances the immunotherapeutic effect. In this context, the modified mRNA of the present invention and the (m)RNA adjuvant can have the same or a similar sequence and even encode the same protein. On the other hand, also a non-coding (m)RNA adjuvant or an (m)RNA adjuvant encoding a different protein or peptide can be combined to achieve the desired adjuvant effect.

In an especially preferred embodiment of the invention, the pharmaceutical composition of the invention includes at least one pharmaceutically acceptable adjuvant and/or carrier. In an especially preferred embodiment of the invention, the composition includes a STING receptor agonist as an adjuvant.

The Stimulator of Interferon Genes (STING) is a transmembrane protein encoded by the TMEM173 gene in humans. The protein is a receptor which works as a cytosolic DNA sensor (CDS) and an adaptor protein in type I interferon signaling. It has been shown to activate downstream transcription factors which are responsible for an antiviral response and an innate immune response against intracellular pathogens. Several viruses have been shown to activate a STING-dependent innate immune response (cf. Wikipedia, "Stimulator of interferon genes"). Molecules which activate the receptor have been described in recent publications Such STING agonists include cyclic dinucleotides (CDNs), like cyclic di-AMP (cGAS) or cyclic GMP-AMP (cGAMP) (Glen N. Barber, Nat Rev Immunol. 2015 December; 15(12);760-770, doi: 10.1038/nri3921; Li et al., Journal of Hematology & Oncology, https://doi.org/10.1186/s13045-019-0721-x). Also DNA molecules or fragments can bind to STING and trigger a response. STING signaling which is triggered by such agonists promotes immunity to DNA viruses and retroviruses, suppresses replication of RNA viruses and can also promote adaptive antitumor immunity. The STING pathway, once triggered, results in an innate immune response that can attract immune cells that phagocytose infected or damaged cells (Glen N. Barber, supra).

Within the context of the present invention, STING agonists like the ones mentioned above, however, also other molecules which activate STING, are the preferred adjuvants to be included in an inventive pharmaceutical composition or vaccine and to be administered together with the inventive modified mRNA in order to elicit an even higher immune response and ensuring immunological memory.

As a further adjuvant, preferably in addition to the STING receptor agonist, the mRNA may be complexed with a cationic or polycationic compound. In a further preferred embodiment, the pharmaceutical composition comprises complexing agents, which protect the mRNA further from degradation. The complexing agent may improve and enhance uptake by cells and concurrent translation into protein. As complexing agents, lipids or polymers can be included in the pharmaceutical composition. In a further preferred embodiment, the pharmaceutical composition can contain the modified mRNA encapsulated in liposomes.

In a further preferred embodiment, the pharmaceutical composition contains cationic lipids. Agents that further improve the delivery of nucleic acids to the cytosols can also be preferably included in the pharmaceutical compositions of the present invention. Such agents can be tailored to the specific route of delivery. In summary, the pharmaceutical compositions of the present invention, while including the inventive modified mRNA as active agent, can include any further substances and any combination of the above mentioned adjuvants, excipients and carriers for improving further the stability of the active substance, enhancing delivery to the cytoplasm of target cells and providing other complementary or synergistic effect.

Still a further subject of the present invention is a kit for preparing a modified mRNA of the present invention. Such kit contains the various substances required for preparing the modified mRNA via in vitro transcription, a fermentation process or a poly(A) polymerase addition reaction. Such kit contains a combination of any of an RNA polymerase and/or poly(A) polymerase, alkyne- or azide-modified nucleotides as well as unmodified nucleotides and optionally further buffer substances and solvents or further substances required for the process. In preferred embodiments, also alkyne- and/or azide-modified functional molecules as well as substances required for performing the click reaction between the modified mRNA and the functional molecules are included. Also, a kit for producing the modified mRNA of the present invention entirely synthetically is a further subject of the present invention. The required substances can be provided in separate containers or can be combined as far as no adverse reaction occurs between such combined substances. As regards the various substances to be included in such kit of parts, it is referred to the above description regarding the modified mRNA of the invention and the processes for preparing such modified mRNA. As far as production of the modified (m)RNA adjuvant is concerned, such kit preferably also contains a cationic or polycationic compound which according to a preferred embodiment is used to form a complex with the (m)RNA. The kits according to the present invention can also contain further substances facilitating the delivery of the modified mRNA of the invention to cells ex vivo or in vivo.

In preferred embodiments, a kit includes at least one modified mRNA of the invention, preferably containing a functional molecule introduced via click reaction, or it provides the modified mRNA and the alkyne- or azide-modified functional molecule and optionally other click reagents in separate containers.

In a further embodiment of the invention, a kit for delivery of the modified mRNA to a patient contains an mRNA and preferably also an (m)RNA adjuvant, both modified according to the present invention. The modified mRNA and the modified (m)RNA adjuvant can be contained in one single container or in separate containers and both can optionally include an alkyne- or azide-modified label or functional molecule either already attached via click reaction or in separate containers for subsequently performing the click reaction. The kit can further contain other pharmaceutically acceptable carriers and adjuvants, again either in separate containers or combined with at least one other constituent of the kit. Especially preferred adjuvants are the above described STING agonists.

It will be apparent to the skilled person that many different combinations of substances can be included in kits, which facilitate the preparation or the use of the modified mRNA of the invention. All embodiments and variations thereof described above in the context of the present invention are also applicable for the kits described here. All suitable combinations of substances are included for the purposes of the present invention.

Further aspects of the present invention are the respective modified mRNAs or pharmaceutical compositions as described above for use in therapeutic and/or prophylactic applications. Preferably, such applications include the use for vaccination against viral infection, especially against Corona virus infections and most preferably against SARS-CoV-2 infection. Prophylactic application in the context of a vaccination is for human and animal use.

Thus, in a further aspect, the invention provides a method for vaccination of an individual against viral infection, especially against a Corona virus infection, and most importantly against the current menace by SARS-CoV-2. The inventive method comprises administering to an individual an effective amount of a modified mRNA or of a pharmaceutical composition/vaccine.

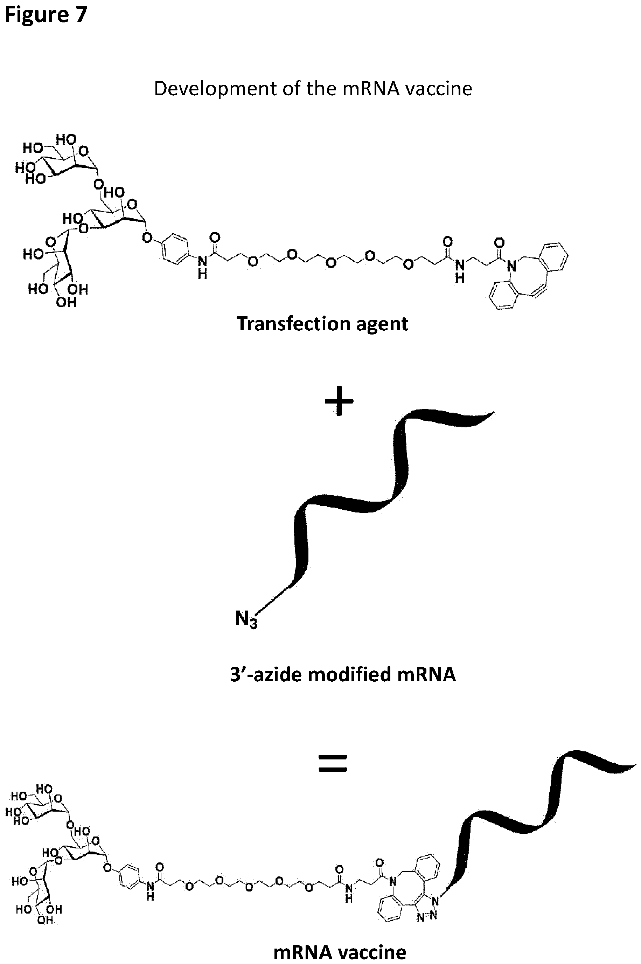

Especially when a combination of a modified mRNA encoding the SARS-CoV-2 protein N and/or protein E, e.g. as disclosed in GenBank entry MN908947.3 for the SARS-CoV-2 isolate Wuhan-Hu-1, or slightly modified sequences, and a STING agonist is used, very effective immunization will be obtained. In this context, the modified mRNA which encodes the viral protein and the STING agonist adjuvant can be either applied together in one single formulation or consecutively in separate dosage forms. In very preferred embodiments of the invention, the mRNA encoding the viral protein(s) is linked to a mannose-click-linker or a GalNAc-click linker as described above and shown in figures 3 and 7. For such mRNA vaccines very efficient transfection of cells and expression of viral proteins could be detected.

Within the context of the application, the administration of the pharmaceutical composition or vaccine can be implemented in all usual manners, especially transdermally or intramuscularly.

In summary, the present invention is based on the inventors' discovery that modified mRNAs, which encode viral proteins or anticancer proteins or parts or epitopes thereof, can be efficiently used to provide antiviral or anticancer vaccines. The modification included with the inventive mRNAs allows for a very specific cell targeting and an efficient delivery or cell transfection leading to a reliable expression of the viral or anticancer protein, even without the usage of LNP. Also, due to the modification, the mRNAs are more stable and, thus, longer available for *in vivo* translation into the encoded protein. In response to this challenge by the viral or anticancer protein, the immune system generates an immune response. The response can also be augmented by addition of an adjuvant, especially a STING receptor agonist. Thus, the present invention provides a solid basis for the development of a toolbox for vaccine production against a vast number of infectious organisms but also for use in immunological cancer treatment methods.

It is to be understood that all information and characterizations provided in this disclosure with regard to the inventive mRNA equally apply in the context of a pharmaceutical composition containing such mRNA and vice versa. The same considerations apply for the processes for production of an inventive mRNA, the uses and methods including the mRNA and the inventive kits.

Thus, even if not explicitly repeated, all embodiments described for one aspect of the invention are applicable to the other embodiments as well.

The figures and the following examples further illustrate the invention:
Fig. 1A shows a depiction from A. Wadhwa et al., Pharmaceutics 2020, 12(2), representing the guiding principle behind mRNA therapy which is based on providing *in vitro*-transcribed mRNA as a carrier of genetic information and delivered by lipid nano particle (LNP), thus allowing the organism to develop its own cure. In vaccination, mRNA which codes for a specific antigen is used to generate an immune response.
Fig. 1B depicts the non-LNP targeted vaccine via modified mRNA.
Fig. 2 is a schematic representation of a Corona Virus SARS-CoV particle (taken from Peiris et al., 2003).
Fig. 3 is a schematic representation of modified vaccine mRNA.
Fig. 4A and 4B show the structures of the transfecting agents (sugar-click-linkers).

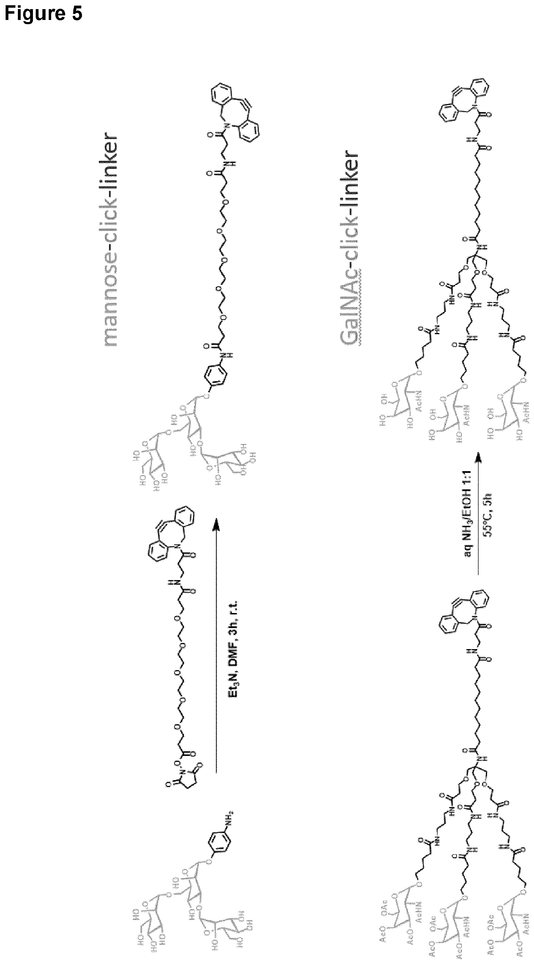

Fig. 5 shows the structure of mannose- and GalNAc-click-linkers and their production.

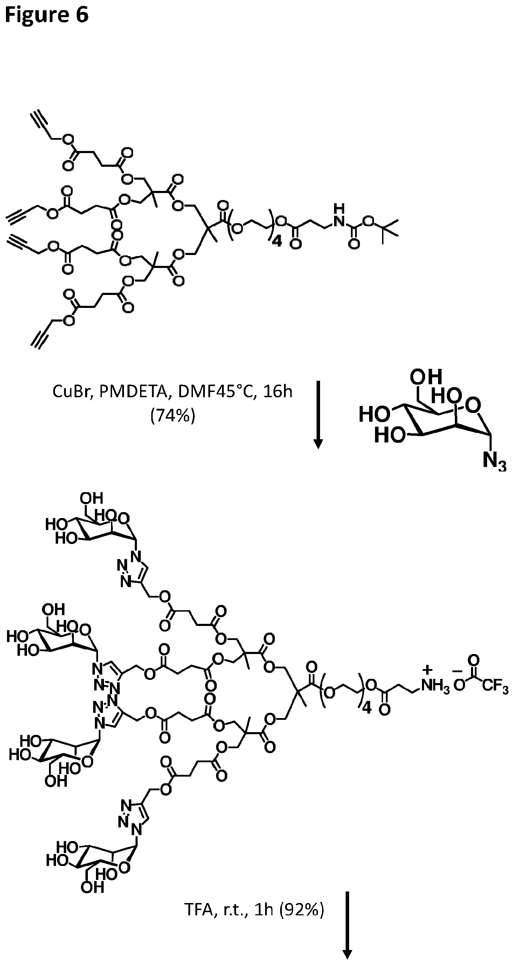

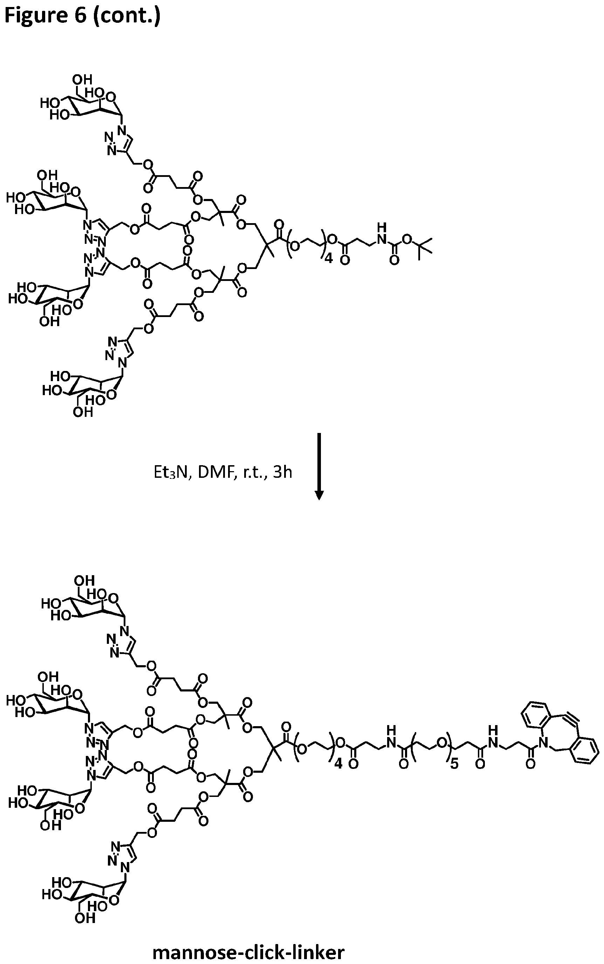

Fig. 6 shows further mannose-click-linkers and their production.
Fig. 7 shows the development of the mRNA vaccine.

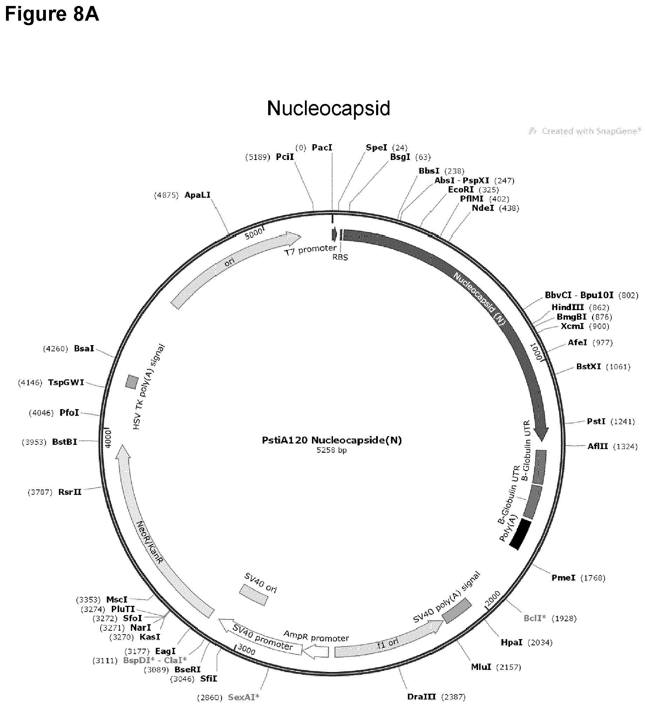

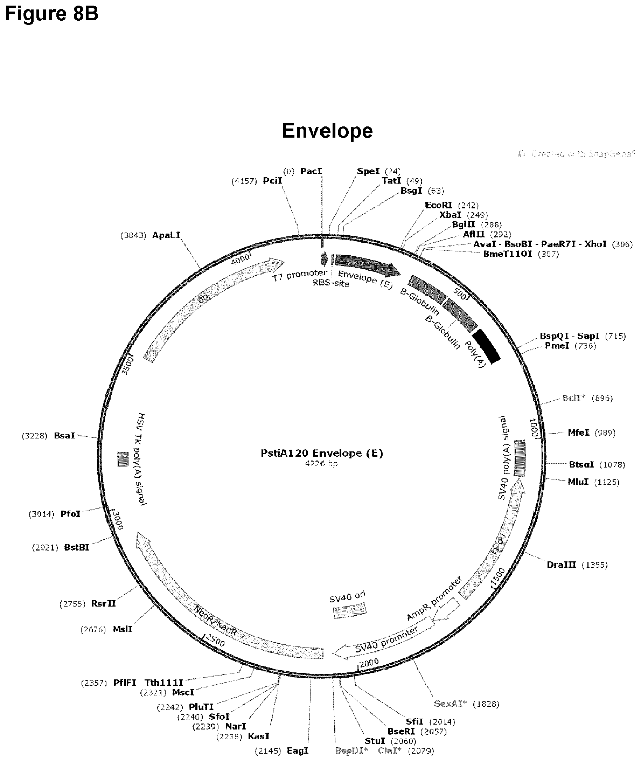

Figs. 8A and 8B show maps of the plasmid PstiA-120 containing the sequences of the nucleocapsid protein or the envelope protein of the SARS-CoV-2.

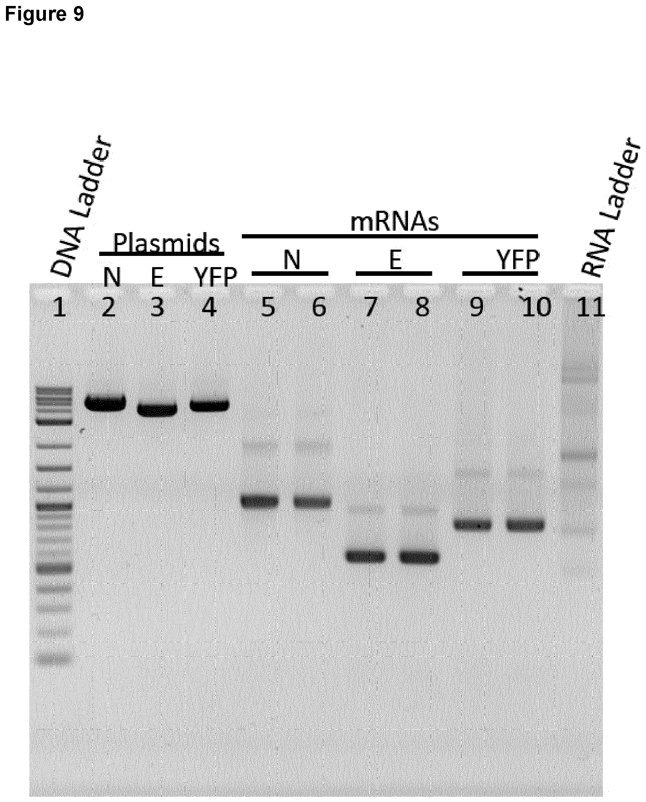

Fig. 9 shows an agarose gel for the analysis of the plasmids and mRNAs of the Envelope (E) and Nucleocapsid (N) protein. Lane 2-4: linearized pStiA120 containing N-E and YFP as ctrl. Lane 5-6: mRNA of N-gene. Lane 7-8: mRNA of E gene. Lane 9-10: mRNA of YFP ctrl.

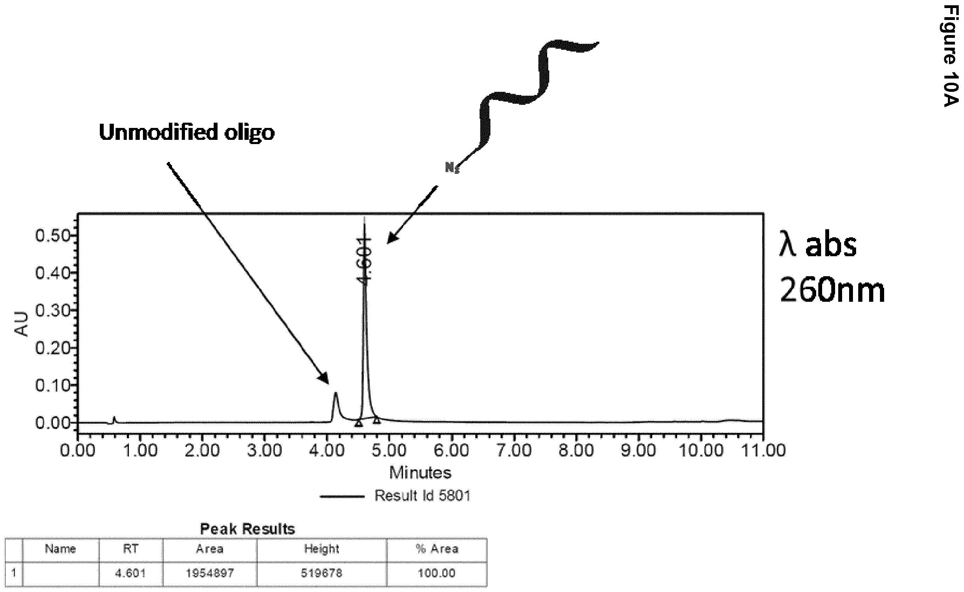

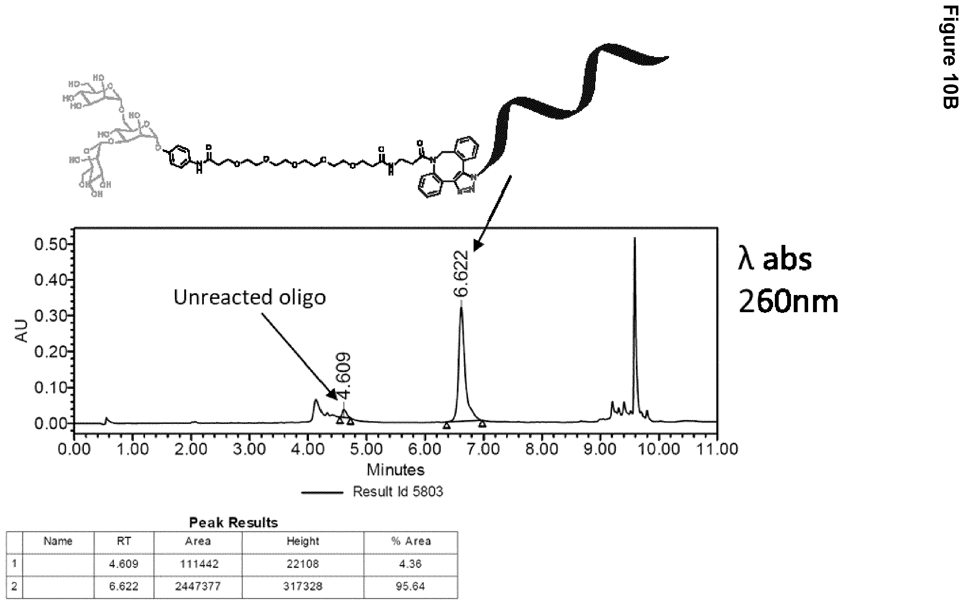

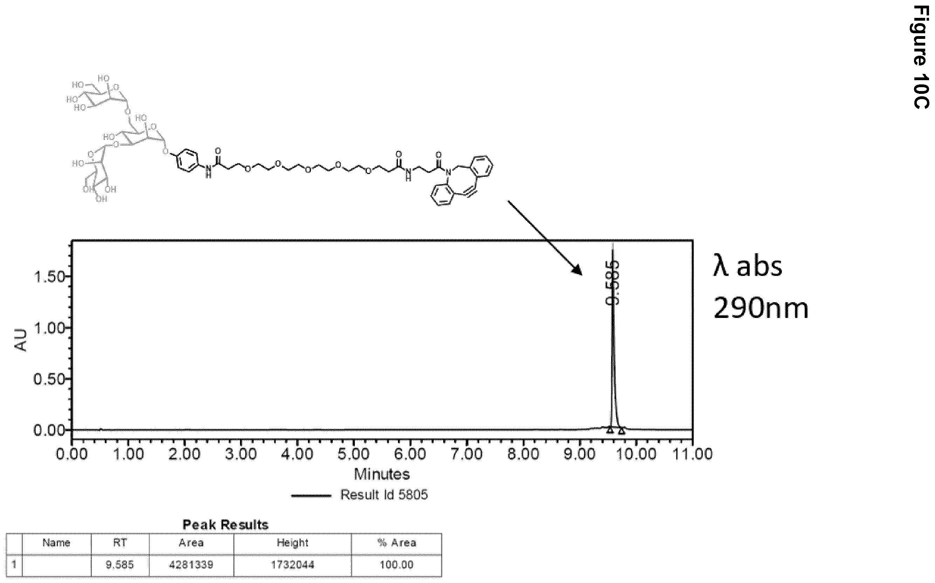

Fig. 10 shows HPL chromatograms of A) Azide modified mRNA. B) Compound 1. C) click product after 3h incubation at room temperature.

### Example 1: Preparation of plasmids encoding N and E protein of SARS-CoV-2

The coding sequences of the Nucleocapsid (N) and Envelope (E) proteins of the novel Coronavirus, SARS-CoV-2, (NCBI GenBank: MN908947.3, Severe acute respiratory syndrome coronavirus 2 isolate Wuhan-Hu-1, complete genome https://www.ncbi.nlm.nih.gov/nuccore/MN908947) were introduced into a vector for the production of the corresponding mRNAs. The vector PstIA-120, already used before (S.Croce et. al., ChemBioChem 2020, 21), was modified by replacing the enhanced green fluorescent protein (eGFP) open reading frame (ORF) with the N and E ORF of SARS-CoV-2 using the Gybosn assembly master mix (New England Biolabs) following the manufacturer's instructions. The sequences of N, E and the 4 primers necessary for the Gybson assembly were ordered from Eurofins Genomics (Table 1).
About 100 ng of the obtained two plasmids (E and N) were transformed into 5-alpha *Escherichia coli* cells (NEB) according to manufacturer's recommendations and then used to inoculate 20 mL LB medium (10 g/L tryptone, 5 g/L yeast extract, 10 g/L NaCl) containing 50 µg/mL kanamycin as a selection marker. The plasmids were then isolated using the plasmid plus midi purification kit (Qiagen) according to manufacturer's instructions. The obtained two plasmids were then sequenced by Sanger sequencing (Eurofins) and the eventual mutations corrected via direct site mutagenesis following the manufacture instruction of Q5 site direct mutagenesis kit. One mutation in the E plasmid and three mutation in the N plasmid were corrected using the primer reported in Table 2.

The obtained plasmids where then linearized using BspQ1 restriction enzyme (NEB) according to manufacturer's recommendations. This results in a linear DNA template that ends directly after the poly A tail encoding sequence. Purity of the DNA template was assessed by nanophotometer measurement (A260/A280 ratio) and agarose gel electrophoresis.

**Table 1: Sequence of the primers used for the Gybosn assembly**

| | |
|---|---|
| **primer 1 plasmid amplification** | TTCGCCCTATAGTGAGTCGTATTAATTAATAAC (SEQ ID NO. 1) |
| **primer 2 plasmid amplification** | TCGAATTGATCCAGATCTTAAGTAAGTAAGC (SEQ ID NO. 2) |
| **Nucleocapside primer 1** | CGACTCACTATAGGGCGAACTAGTAAGCAAGGAGG CGT (SEQ ID NO. 3) |
| **Nucleocapside primer 2** | ACTTAAGATCTGGATCAATTCGATTAGGCCTGAGTTG AGTCAG (SEQ ID NO. 4) |
| **Envelope primer 1** | CGACTCACTATAGGGCGAACTAGTAAGCAAGGAGG CG (SEQ ID NO. 5) |
| **Envelope primer 2** | ACTTAAGATCTGGATCAATTCGATTAGACCAGAAGAT CAGGAACT (SEQ ID NO. 6) |
| **Nucleocapside ORF** | |
| CTAGTAAGCAAGGAGGCGTGCAGATGTCTGATAATGGACCCCAAAATCA GCGAAATGCACCCCGCATTACGTTTGGTGGACCCTCAGATTCAACTGGC AGTAACCAGAATGGAGAACGCAGTGGGGCGCGATCAAAACAACGTCGG CCCCAAGGTTTACCCAATAATACTGCGTCTTGGTTCACCGCTCTCACTC AACATGGCAAGGAAGACCTTAAATTCCCTCGAGGACAAGGCGTTCCAAT TAACACCAATAGCAGTCCAGATGACCAAATTGGCTACTACCGAAGAGCT ACCAGACGAATTCGTGGTGGTGACGGTAAAATGAAAGATCTCAGTCCAA GATGGTATTTCTACTACCTAGGAACTGGGCCAGAAGCTGGACTTCCCTA TGGTGCTAACAAAGACGGCATCATATGGGTTGCAACTGAGGGAGCCTT GAATACACCAAAAGATCACATTGGCACCCGCAATCCTGCTAACAATGCT GCAATCGTGCTACAACTTCCTCAAGGAACAACATTGCCAAAAGGCTTCT ACGCAGAAGGGAGCAGAGGCGGCAGTCAAGCCTCTTCTCGTTCCTCAT CACGTAGTCGCAACAGTTCAAGAAATTCAACTCCAGGCAGCAGTAGGG GAACTTCTCCTGCTAGAATGGCTGGCAATGGCGGTGATGCTGCTCTTGC TTTGCTGCTGCTTGACAGATTGAACCAGCTTGAGAGCAAAATGTCTGGT AAAGGCCAACAACAACAAGGCCAAACTGTCACTAAGAAATCTGCTGCTG AGGCTTCTAAGAAGCCTCGGCAAAAACGTACTGCCACTAAAGCATACAA | |
| TGTAACACAAGCTTTCGGCAGACGTGGTCCAGAACAAACCCAAGGAAAT TTTGGGGACCAGGAACTAATCAGACAAGGAACTGATTACAAACATTGGC CGCAAATTGCACAATTTGCCCCCAGCGCTTCAGCGTTCTTCGGAATGTC GCGCATTGGCATGGAAGTCACACCTTCGGGAACGTGGTTGACCTACAC AGGTGCCATCAAATTGGATGACAAAGATCCAAATTTCAAAGATCAAGTCA TTTTGCTGAATAAGCATATTGACGCATACAAAACATTCCCACCAACAGAG CCTAAAAAGGACAAAAAGAAGAAGGCTGATGAAACTCAGCCTTACCGCA GAGACAGAAGAAACAGCAAACTGTGACTCTTCTTCCTGCTGCAGATTTG GATGATTTCTCCAAACAATTGCAACAATCCATGAGCAGTGCTGACTCAA CTCAGGCCTAA (SEQ ID NO. 7) | |

| **Envelope ORF** | |
|---|---|
| CTAGTAAGCAAGGAGGCGTGCAGATGTACTCATTCGTTTCGGAAGAGAC AGGTACGTTAATAGTTAATAGCGTACTTCTTTTTCTTGCTTTCGTGGTATT CTTGCTAGTTACACTAGCCATCCTTACTGCGCTTCGATTGTGTGCGTACT GCTGCAATATTGTTAACGTGAGTCTTGTAAAACCTTCTTTTTACGTTTACT CTCGTGTTAAAAATCTGAATTCTTCTAGAGTTCCTGATCTTCTGGTCTAA (SEQ ID NO. 8) | |

**Table 2: Sequences of the primers used for the site directed mutagenesis.**

| | |
|---|---|
| **Envelope Sdm 1 Fwd** | CAGTACGCACACAATCGA (SEQ ID NO. 9) |
| **Envelope Sdm 1 Rev** | CTGCAATATTGTTAACGTGAG (SEQ ID NO. 10) |
| **Nucleocapsid 1 Fwd** | AGGTAGTAGAAATACCATCTTGC (SEQ ID NO. 11) |
| **Nucleocapsid 1 Rev** | AGGAACTGGGCCAGAAG (SEQ ID NO. 12) |
| **Nucleocapsid 2 Fwd** | TGAGTTTCATCAGCCTTCTT (SEQ ID NO. 13) |
| **Nucleocapsid 2 Rev** | AGCCTTACCGCAGAGAC (SEQ ID NO. 14) |
| **Nucleocapsid 3 Fwd** | TACCGACGAGCTACCAGACGA (SEQ ID NO. 15) |
| **Nucleocapsid 3 Rev** | GTAGCCAATTTGGTCATCTGGACT (SEQ ID NO. 16) |

### Example 2: Production of mRNA encoding proteins N and E

mRNA production was performed by *in vitro* transcription from the linear version of the plasmids obtained in Example 1, using T7 RNA polymerase.

In a 50 µL final reaction volume 20 units of T7 RNA polymerase (THERMO FISHER), 1 µg of linear template DNA and several nucleotides were combined in transcription buffer (40 mM Tris-HCI, pH 7.9, 6 mM MgCl₂, 4 mM spermidine, 10 mM DTT).

**Table 3: Final concentrations of nucleotide mixtures used for the production of (modified) mRNAs.**

| **ARCA c [mM]** | **ATP c [mM]** | **CTP c [mM]** | **GTP c [mM]** | **UTP c [mM]** | **□UTP c [mM]** |
|---|---|---|---|---|---|
| 4.00 | 1.50 | 1.25 | 1.00 | 1.25 | 1.25 |

Transcription reactions were incubated for 2 hours at 37 °C and then 2 units of DNAse I (THERMO FISHER) were added and incubated for 15 minutes at 37 °C to digest the DNA template and make its removal easier. The mRNA was purified by a spin column method according to manufacturer's instruction (QIAGEN).

Out of 1 µg of plasmid, about 12 µg of mRNA are produced.
The mRNAs are composed of the 5' untranslated region (UTR) with the ribosome binding site (rbs), the open reading frame (ORF) of the proteins, a 3' UTR consisting of two repetitive sequences of the human beta globulin 3' UTR and a poly(A) tail. The N mRNA is composed of 1730 nt, the E mRNA of 700 nt. After purification using spin column methods (Quiagen), the quality of the mRNA is subsequently determined by nanodrop and agarose gel analysis. The results are shown in Fig. 9.

### Example 3: 3' Azide labelling and mannose modification of mRNA

In a 25 µL reaction volume 600 units of yeast poly(A) polymerase, 10 µg of mRNA and 3'-Azido-2',3'-ddATP (0.5 mM final concentration) were combined in reaction buffer (20 mM Tris-HCI, pH 7.0, 0.6 mM MnCl₂, 20 µM EDTA, 200 µM DTT, 10 µg/mL acetylated BSA, 10 % glycerol). The mixture was incubated for 20 minutes at 37 °C. mRNA was purified by a spin column method according to manufacturer's instruction (Qiagen).

### Mannose based transfection agent synthesis

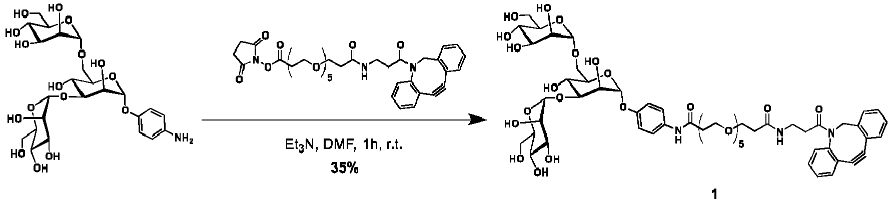

**Compound 1.** 4-Aminophenyl-3,6-di-O-(α-D-mannopyranosyl)-α-D-mannopyranoside (10 mg, 16.8 µmol, 1 eq) was dissolved in 100 µL of DMF. DBCO-PEG5-NHS (23.3 mg, 33.6 µmol, 2 eq) and Et₃N (7 µL, 50.7 µmol, 3 eq) were added and the solution was stirred at room temperature for 1h. The solvent was removed *in vacuo* and the crude material was purified by reversed phase preparative HPLC to afford **1** in 35% yield (7 mg, 5.96 µmol).

### Model system for the SPAAC

In order to prove the efficiency of the Strain promoted Azide-Alkyne cycloaddition a short RNA oligonucleotide (31 mer) containing a 5' azide was used as model template. 4 µg of RNA oligo and 2 nmol of compound 1 were combined in a total reaction volume of 30 µL and incubated at room temperature for 3h and the resulting products were analysed by HPLC (Fig. 10).

### Production of mRNA vaccine by SPAAC

When click labeling was performed, 10 µg of purified azide mRNA, 2 nmol Compound 1, were combined in a total reaction volume of 30 µL. The reaction mixture was incubated at room temperature overnight and then cleaned using a spin column method according to manufacturer's instruction (Qiagen).

Fig. 10 shows the HPL Chromatograms of the SpAAC modification reaction. In part A, the higher peak represents azide modified mRNA, the smaller peak represents the unmodified oligonucleotide. The peak in part B represents the DBCO 3-mannose structure and part C shows the unreacted oligonucleotide, the clicked product after 2 hours incubation at room temperature and residual DBCO 3-mannose structure. The HPLC chromatograms show more than 95% conversion.

## Claims

1. Modified messenger RNA (mRNA) which encodes within its ORF an antigen such as a viral protein, an immunogenically active part of such viral protein or an anticancer protein or epitope **characterized in that** it contains at least one of an alkyne- or azide-modification in at least one nucleotide.

2. Modified mRNA according to claim 1, wherein the viral protein is a Corona virus protein, preferably a Corona virus nucleoprotein, most preferably one or both of nucleoproteins N and envelope protein E of SARS-CoV-2.

3. Modified mRNA according to claim 1 or 2, comprising a 5'-cap structure, a 5'-untranslated region (5'-UTR), an open reading frame region (ORF), a 3'-untranslated region (3'-UTR) and a poly(A) tail region, **characterized in that** it contains at least one of an alkyne- or azide-modification in at least one nucleotide within at least one of the ORF, the 5'-UTR, the 3'-UTR and the poly(A) tail region.

4. Modified mRNA according to anyone of claims 1 to 3, **characterized in that** it contains modified nucleotides in
a) the ORF and the UTRs,
b) the ORF, the UTRs and the poly(A) tail, or
c) only the poly(A) tail.

5. Modified mRNA according to anyone of claims 1 to 4, wherein at least one of the four standard types of nucleotides (AMP, CMP, GMP, UMP) are partly or completely modified, preferably ethynyl- or azido-modified at uracil or adenine.

6. Modified mRNA according to anyone of claims 1 to 5, wherein at least one nucleotide is alkyne-modified and at least one nucleotide is azide-modified.

7. Modified mRNA according to any one of the preceding claims, wherein at least one of the four standard types of nucleotides is present in modified form compared to the non-modified form in a ratio of 1:100 to 10:1, preferably 1:10 to 1:10 or 1:1.

8. Modified mRNA according to any one of the preceding claims, **characterized in that** it contains otherwise modified natural or artificial nucleotides, preferably pseudouridine or N1-methylpseudouridine.

9. Modified mRNA according to any one of the preceding claims, wherein the modified mRNA contains functional molecule(s) introduced via a click reaction of the modified mRNA with a correspondingly modified alkyne- or azide-containing functional molecule(s).

10. Modified mRNA according to claim 9, wherein the functional molecule is a substance, which increases the half-life of the mRNA, enhances expression of the mRNA, acts as an adjuvant or is a cell-specific targeting group or ligand.

11. Modified mRNA according to claim 9 or 10, wherein the functional molecule is a sugar moiety, a fatty acid moiety, a cell-type specific antibody or fragment of such antibody, especially an anti-CD20 or anti-CD19 antibody or antibody fragment.

12. Modified mRNA according to claim 11, wherein the sugar moiety includes mannose and/or N-acetyl galactosamine.

13. Modified mRNA according to anyone of claims 9 to 12, wherein the functional molecule comprises linker molecules comprising one or more attached sugar moieties.

14. Modified mRNA according claim anyone of claims 9 to 13, wherein the functional molecules target lymphocytes, in particular MHC1 peptide presenting cells, such as mast cells.

15. Modified RNA containing at least one alkyne- or azide- modification in at least one nucleotide or modified mRNA according to anyone of claims 1 to 14, which is complexed with a cationic or polycationic compound.

16. Process for the production of the modified mRNA according to any one of the preceding claims, wherein the process comprises in vitro transcribing mRNA from a DNA template or alternatively performing a fermentation process using prokaryotic or eukaryotic host cells to express a DNA template contained in an expression vector wherein the process is performed in the presence of an RNA polymerase and a nucleotide mixture containing the four standard types of nucleotides required for mRNA transcription, in which nucleotide mixture at least a part of at least one of the four types of nucleotides is modified to contain an alkyne- or azide-modification.

17. Process for the production of a modified mRNA containing an alkyne- or an azide-modification at the poly(A) tail, wherein the process comprises performing a poly(A) polymerase addition reaction at the poly(A) tail on an mRNA in the presence of ATP, wherein ATP is at least partly alkyne- or azide-modified at the adenosine.

18. Process according to claims 16 or 17, further comprising adding one or more correspondingly alkyne- or azide-modified functional molecule(s) under conditions to perform a click reaction to produce a modified mRNA according to anyone of claims 9 to 13.

19. Pharmaceutical composition, comprising a modified mRNA according to anyone of claims 1 to 15 as an active agent, optionally in combination with a pharmaceutically acceptable adjuvant or excipient and/or contained in pharmaceutically acceptable carrier.

20. Pharmaceutical composition according to claim 19, wherein the adjuvant is a STING receptor agonist.

21. Modified mRNA according to anyone of claims 1 to 5 or a pharmaceutical composition according to claim 19 or 20 for use in mRNA based therapeutic and/or prophylactic applications.

22. Modified mRNA according to anyone of claims 1 to 15 or pharmaceutical composition according to claim 19 or 20, for use in therapeutic and / or prophylactic application according to claim 21, wherein the therapeutic and/or prophylactic application comprises vaccination against viral infection, especially against Corona virus infection, and most preferably against SARS-CoV-2 infection.

23. Modified mRNA according to anyone of claims 1 to 15 or pharmaceutical composition according to claim 19 or 20, for use in prophylactic application according to claims 21 in a human or an animal.

24. A kit for production and/or delivery of a modified mRNA according to anyone of claims 1 to 15.

25. Method for vaccination of an individual against viral infection, especially against Corona virus infection, and most preferably against SARS-CoV-2 infection, which method comprises administering to such individual an effective amount of a modified mRNA according to anyone of claims 1 to 15 or of a pharmaceutical composition according claim 19 or 20.
